# EUROPEAN PATENT SPECIFICATION

(11) **EP 0 972 770 B1**
(45) Date of publication and mention of the grant of the patent: **19.09.2001**
(21) Application number: 99305172.1
(22) Date of filing: 30.06.1999
(51) Int. Cl.: C07D 239/42, C07D 239/26, C07D 239/46, C07D 239/34, A01N 43/54

(54) **Acaricidal and insecticidal substituted pyrimidines and a process for the preparation thereof**
Akarizid und insektizid wirksame, substituierte Pyrimidine und Verfahren zu ihrer Herstellung
Pyrimidines substituées à activité acaricide et insecticide et procédé de leur préparation

(30) Priority: 14.07.1998 US 115309
(43) Date of publication of application: 19.01.2000
(73) Proprietor: BASF AKTIENGESELLSCHAFT, 67056 Ludwigshafen (DE)
(72) Inventor: Cuccia, Salvatore John, Mercerville, New Jersey 08648 (US); Wood, William Wakefield, Pennington, New Jersey 08534 (US); Buckwalter, Brian Lee, Yardley, Pennsylvania 19067 (US); Chiarello, John Francis, Newtown, Pennsylvania 18940 (US); Fleming, Linda Barbara, Ewing, New Jersey 08638 (US)
(74) Representative: Köster, Reinhold, Dr.

(56) References cited:
- WO-A-98/54154
- DE-A- 4 327 596
- DE-A- 19 602 095
- US-A- 5 707 995

## Description

### BACKGROUND OF THE INVENTION

A class of pesticidal diaryl pyrimidine compounds is disclosed in U. S. 5,707,995. These compounds all contain 4,6-bis-aryl-pyrimidines wherein the aromatic groups are attached via two linking groups which are identical to one another. The present invention relates to a class of 4,6-bis-aryl-pyrimidines linked by different functional groups which has surprisingly been found to have acaricidal and insecticidal activity.

### SUMMARY OF THE INVENTION

The present invention provides compounds of Formula I 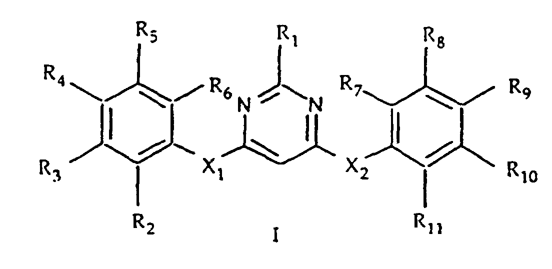 wherein
R₁ is hydrogen, (C₁₋₆)alkylthio, halo(C₁-6)alkylthio (C₁-₆)alkylsulphinyl, halo(C₁-₆)alkylsulphinyl, (C₁₋₆)alkylsulphonyl, halo(C₁-₆)alkylsulphonyl or NR₁₂R₁₃;
R₁₂ and R₁₃ are each independently hydrogen, (C₁-₆)alkyl, or halo(C₁-₆)alkyl;
R₂, R₃, R₄, R₅, R₆, R₇, R₈, R₉ and R₁₁ are each independently selected from hydrogen, halogen, cyano, nitro, (C₁-₆)alkyl, halo(C₁-₆)alkyl, (C₁-₆)alkoxy, halo (C₁₋₆)alkoxy, (C₁-₆)alkoxy(C₁-₆)alkyl, halo(C₁-₆)alkoxy(C₁-₆)alkyl, (C₁-₆)alkylthio, halo(C₁-₆)alkylthio, (C₁-₆)alkyl sulphinyl, halo(C₁₋₆)alkylsulphinyl, (C₁₋₆)alkylsulphonyl, halo(C₁₋₆)alkylsulphonyl, amino, (C₁-₆)alkylamino, di(C₁-₆)alkylamino, halo(C₁-6)alkylamino, (C₁-₆)alkylhaloalkyl (C₁-₆)amino, dihalo(C₁₋₆)alkylamino, (C₁-₆)alkyloxy-carbonyl, halo(C₁-6)alkyloxycarbonyl, (C₂-₆)alkenyl, halo(C₂-₆)alkenyl, (C₂-₆)alkynyl, halo(C₂-₆)alkynyl;
R₁₀ is halogen, halo(C₁-₆)alkyl, halo(C₂-₆)alkenyl or halo(C₂-₆)alkynyl;
and X₁ and X₂ are independently selected from the group consisting of NR₁₄, NR₁₅, O, CH₂, CR₁₈R₁₉, CO, C=NOR₂₀; wherein R₁₄ is (C₁-₆)alkyl;
R₁₅ is H, (C₁-₆)alkyl or CH(OR₁₆) (OR₁₇);
R₁₆ and R₁₇ are each independently (C₁-₆)alkyl;
R₁₈ and R₁₉ each independently selected from H, (C₁-₆)alkyl, (C₂-₆)alkenyl and (C₂-₆)alkynyl which are all optionally substituted by one or more halogens which are the same or different, or R₁₈ and R₁₉ may together form a carbocyclic ring which is optionally substituted by one or more halogens which are the same or different;
R20 is (C₁₋₆)alkyl ;
with the proviso that X₁ cannot be the same as X₂.

Also provided are a process for the preparation of the formula I compound and a method for the control of acarina and insects therewith.

### DETAILED DESCRIPTION OF THE INVENTION

Preferred compounds of the present invention include compounds of Formula I 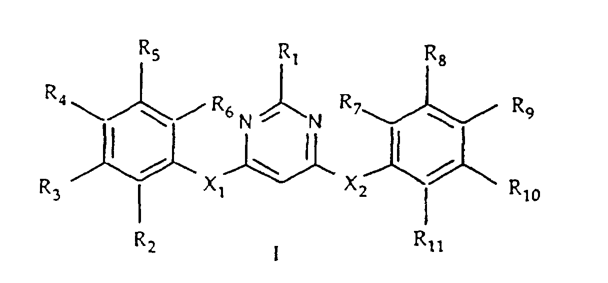 wherein
R₁ is hydrogen, (C₁₋₆)alkylthio, halo(C₁-6)alkylthio (C₁-₆)alkylsulphinyl, halo(C₁-₆)alkylsulphinyl, (C₁₋₆)alkylsulphonyl, halo(C₁-₆)alkylsulphonyl or NR₁₂R₁₃;
R₁₂ and R₁₃ are each independently hydrogen, (C₁-₆)alkyl, or halo(C₁-₆)alkyl;
R₂, R₃, R₄, R₅, R₆, R₇, R₈, R₉ and R₁₁ are each independently selected from hydrogen, halogen, cyano, nitro, (C₁-₆)alkyl, halo(C₁-₆)alkyl, (C₁-₆)alkoxy, halo (C₁-₆)alkoxy, (C₁-₆)alkoxy(C₁-₆)alkyl, halo(C₁-₆)alkoxy(C₁-₆)alkyl, (C₁-₆)alkylthio, halo(C₁-₆)alkylthio, (C₁-₆)alkyl sulphinyl, halo(C₁-₆)alkylsulphinyl, (C₁-₆)alkylsulphonyl, halo(C₁-₆)alkylsulphonyl, amino, (C₁-₆)alkylamino, di(C₁-₆)alkylamino, halo(C₁-₆)alkylamino, (C₁-₆)alkylhalo(C₁-₆)alkylamino, dihalo(C₁-₆)alkylamino, (C₁-₆)alkyloxycarbonyl, halo(C₁-₆)alkyloxycarbonyl, (C₂-₆)alkenyl, halo(C₂-₆)alkenyl, (C₂-₆)alkynyl, halo(C₂-₆)alkynyl;
R₁₀ is halogen, halo(C₁-₆)alkyl, halo(C₂-₆)alkenyl or halo(C₂-₆)alkynyl ;
   and a) one of X₁ and X₂ is NR₁₄ and the other is O or S, wherein R₁₄ is (C₁-₆)alkyl
   or b) one of X₁ and X₂ is NR₁₅ and the other is CH₂, wherein R₁₅ is H, (C₁-₆)alkyl or CH(OR₁₆)(OR₁₇) and R₁₆ and R₁₇ are each independently (C₁-₆)alkyl ;
   or c) one of X₁ and X₂ is CR₁₈R₁₉ or CO and the other is O, wherein R₁₈ and R₁₉ each independently selected from H, (C₁-₆)alkyl, (C₂-₆)alkenyl and (C₂-₆)alkynyl which are all optionally substituted by one or more halogens which are the same or different, or R₁₈ and R₁₉ may together form a carbocyclic ring which is optionally substituted by one or more halogens which are the same or different
   or d) one of X₁ and X₂ is C=NOR₂₀ and the other is CH_{2,} wherein R20 is (C₁-₆)alkyl.

4-[m-(trifluoromethyl)benzyl]-6-(α,α,α-trifluoro-N-methyl-m-toluidino)pyrimidine is a less preferred compound of the invention.

When used herein as a definition or part of a definition the term halogen or halo refers to fluorine, chlorine, bromine and iodine; preferred halogens are fluorine and chlorine; more preferred is fluorine.

When used herein as a definition or part of a definition the term alkyl is a straight or branched chain group such as methyl, ethyl, n-propyl, i-propyl, n-butyl, s-butyl, t-butyl, n-pentyl, 2-pentyl, 3-pentyl and n-hexyl. Preferred alkyl groups are methyl, ethyl, n-propyl, i-propyl, n-butyl, s-butyl, t-butyl and n-pentyl; more preferred alkyl groups are methyl and ethyl.

When used herein as a definition or part of a definition the term alkenyl is a straight or branched chain group such as vinyl, 1-propenyl, 2-propenyl, 1-butenyl, 2-butenyl, 3-butenyl, pentenyls and hexenyls.

When used herein as a definition or part of a definition the term alkynyl is a straight or branched chain group such as ethynyl, 1-propynyl, 2-propynyl, 1-butynyl, 2-butynyl and 3-butynyl.

R₁₀ is preferably chlorine, halo(C₁-₄)alkyl or halovinyl; more preferably chlorine or a (C₁-₄)alkyl or vinyl group substituted by one or more fluorine or chlorine atoms; most preferably CF₃ or CF₂CF₃.

R₃ is preferably halogen, halo(C₁-₆)alkyl, halo(C₂-₆)alkenyl or halo(C₂-₆)alkynyl; more preferably R₃ is halo, halo(C₁-₄)alkyl or halovinyl; most preferably chlorine or a (C₁-₄)alkyl or vinyl group substituted by one or more fluorine or chlorine atoms; particular examples of R₃ are chlorine, CF₃ or CF₂CF₃.

R₄ and R₉ are each preferably selected from hydrogen, halogen, cyano, nitro, (C₁-₆)alkyl, halo(C₁-₆)alkyl, (C₁-₆)alkoxy, halo (C₁-₆)alkoxy, (C₁-₆)alkoxy(C₁₋₆)alkyl, halo(C₁-₆)alkoxy(C₁-₆)alkyl, (C₁-₆)alkylthio, (C₂-₆)alkenyl, halo(C₂-₆)alkenyl, (C₂-₆)alkynyl, halo(C₂-₆)alkynyl; more preferably hydrogen or a halogen, most preferably fluorine or chlorine.

R₂, R₅, R₆, R₇, R₈ and R₁₁ are each preferably selected from hydrogen, halogen, cyano, nitro, (C₁-₆)alkyl, halo(C₁-₆)alkyl, (C₁-₆)alkoxy, halo (C₁-₆)alkoxy, (C₁-₆)alkoxy(C₁-₆)alkyl, halo(C₁-₆)alkoxy(C₁-₆)alkyl, (C₁-₆)alkylthio, (C₂-₆)alkenyl, halo(C₂-6)alkenyl, (C₂-₆)alkynyl, halo(C₂-₆)alkynyl; more preferably each is hydrogen or a halogen, most preferably hydrogen.

R₁₂ and R₁₃ are each preferably selected from methyl, ethyl, propyl or butyl, more preferably they are both methyl. R₁₄, R₁₅, R₁₆ and R₁₇ are each preferably methyl, ethyl, propyl or butyl, more preferably methyl or ethyl. Preferred compounds being those in which R₁₆ and R₁₇ are identical.

R₁₈ and R₁₉ are preferably methyl, ethyl, propyl, butyl or together form a cyclopropyl, cyclobutyl or cyclopentyl ring. More preferably both R₁₂ and R₁₃ are methyl or they together form a cyclopropyl ring.

A preferred group of compounds are those wherein X₁ is NR₁₄ and X₂ is O. Preferred embodiments within this group are those wherein R₃, R₄ and R₉ are independently selected from hydrogen, halo, halo(C₁-₆)alkyl, halo(C₂-6)alkenyl or halo(C₂-₆)alkynyl. Preferred compounds within this group are those wherein R₁₀ is halo(C₁-₆)-alkyl and R₉ is hydrogen or a halogen, particularly compounds wherein R₁₀ is CF₃ and R₉ is hydrogen, chlorine or fluorine. More preferred compounds within this group are those wherein R₄ is hydrogen or a halogen and R₃ is halo(C₁-₆)alkyl, particularly where R₄ is hydrogen, chlorine or fluorine and R₃ is CF3. Most preferred compounds within this group are those wherein R₃ and R₁₀ are CF₃ and R₄ and R₉ are fluorine or chlorine, preferably fluorine. Also preferred are compounds wherein R₃ and R₄ are both halogens, particularly those compounds wherein R₃ and R₄ are both chlorine. In these embodiments R₂, R₅, R₆, R₇, R₈ and R₁₁ are preferably hydrogen and R₁₄ is preferably methyl or ethyl, more preferably methyl.

Preferred compounds within this group are :
4-[(α,α,α,4-Tetrafluoro-N-methyl-m-toluidino)-6-[(α,α,α,4-tetrafluoro-m-tolyl)oxy]pyrimidine ;
4-(α,α,α,-Trifluoro-N-methyl-m-toluidino)-6-[(α,α,α-trifluoro-m-tolyl)oxy]pyrimidine ;
4-[(α,α,α,4-Tetrafluoro-n-methyl-m-toluidino)-6-[(α,α,α,4-tetrafluoro-m-tolyl)oxy]pyrimidine ;
4-(3,4-Dichlorophenoxy)-6-(α,α,α-trifluoro-N-methyl-m-toluidino)pyrimidine;
4-[(4-Chloro-α,α,α-trifluoro-m-tolyl)oxy]-6-(α,α,α-trifluoro-N-methyl-m-toluidino)pyrimidine;
4-(α,α,α,4-Tetrafluoro-N-methyl-m-toluidino)-6-[(α,α,α-trifluoro-m-tolyl)oxy]pyrimidine;
4-[(4-Chloro-α,α,α,-trifluoro-m-tolyl)oxy]-6-(α,α,α,4-tetrafluoro-N-methyl-m-toluidino)pyrimidine;
4-(4-Chloro-α,α,α,-trifluoro-N-methyl-m-toluidino)-6-[(α,α,α,4-tetrafluoro-m-tolyl)oxy]pyrimidine ;
4-(3,4-Dichloro-N-methylanilino)-6-[(α,α,α,-trifluoro-m-tolyl)oxy]pyrimidine;
4-(3,4-Dichloro-N-methylanilino)-6-[(α,α,α,-4-tetrafluoro-m-tolyl)oxy]pyrimidine; and
4-(3,4-Dichloro-N-methylanilino)-6-(3,4-dichlorophenoxy)pyrimidine.

A further preferred group of compounds are those wherein X₁ is CH₂ and X₂ is NR₁₅. Preferred embodiments within this group are those wherein R₃, R₄ and R₉ are independently selected from hydrogen, halo, halo(C₁-₆)alkyl, halo(C₂-₆)alkenyl or halo(C₂-₆)alkynyl. Preferred compounds within this group are those wherein R₁₀ is halo(C₁-₆)alkyl and R₉ is hydrogen or a halogen, particularly compounds wherein R₁₀ is CF₃ and R₉ is hydrogen, chlorine or fluorine. More preferred compounds within this group are those wherein R₃ is halo(C₁-₆)alkyl and R₄ is hydrogen or a halogen, particularly where R₃ is CF₃ and R₄ is hydrogen, chlorine or fluorine. Most preferred compounds within this group are those wherein R3 and R₁₀ are CF₃ and R₄ and R₉ are fluorine or chlorine, preferably fluorine. Also preferred are compounds wherein R₃ and R₄ are both halogens, particularly those compounds wherein R₃ and R₄ are both chlorine. In these embodiments R₂, R₅, R₆, R₇, R₈ and R₁₁ are preferably hydrogen and R₁₅ is preferably hydrogen, methyl or ethyl, more preferably methyl.

Preferred compounds within this group are:
4-(α,α,α,4-Tetrafluoro-m-toluidino)-6-[m-(trifluoromethyl)benzyl]pyrimidine hydrochloride;
4-(3,4-Dichlorobenzyl)-6-(α,α,α,4-tetrafluoro-m-toluidino)pyrimidine hydrochloride;
4-(4-Chloro-α,α,α,-trifluoro-m-toluidino)-6-[m-(trifluoromethyl)benzyl]pyrimidine hydrochloride;
4-[m-(Trifluoromethyl)benzyl]-6-(α,α,α,-trifluoro-m-toluidino)pyrimidine hydrochloride;
4-(α,α,α,4-Tetrafluoro-N-methyl-m-toluidino)-6-[m-trifluoromethyl)benzyl]pyrimidine;
4- (4-Chloro-α,α,α,-trifluoro-N-methyl-m-toluidino)-6-(3,4-dichlorobenzyl)pyrimidine;
4-(3,4-Dichlorobenzyl)-6-(α,α,α,4-tetrafluoro-N-methyl-m-toluidino)pyrimidine;
4-(4-Chloro-α,α,α,-trifluoro-m-toluidino)-6-(3,4-dichlorbenzyl)pyrimidine;
4-(3,4-Dichlorobenzyl)-6-[N-diethoxymethyl)-α,α,α,4-tetrafluoro-m-toluidino]pyrimidine;
4-(4-Chloro-α,α,α,-trifluoro-N-methyl-m-toluidino)-6-[m-(trifluoromechyl)benzyl]pyrimidine;
4-(3,4-Dichlorobenzyl)-6-(α,α,α-trifluoro-m-toluidino)pyrimidine;
6-(α,α,α,4-Tetrafluoro-m-toluidino)-4- [4-fluoro-3-(trifluoromethyl)benzyl]pyrimidine;
4-[4-Fluoro-3-(trifluoromethyl)benzyl]-6-(α,α,α,4-tetrafluoro-N-methyl-m-toluidino)pyrimidine;
4-(4-chloro-α,α,α,-trifluoro-m-toluidino)-6-[4-fluoro-3-(trifluoromethyl)benzyl]pyrimidine; and
4-(4-chloro-α,α,α,-trifluoro-N-methyl-m-toluidino)-6-[4-fluoro-3-(trifluoromethyl)benzyl]pyrimidine.

A further preferred group of compounds are those wherein X₁ is CR₁₈R₁₉ or CO and X2 is O. Preferred embodiments within this group are those wherein R₃, R₄ and R₉ are independently selected from hydrogen, halogen, halo(C₁-₆)alkyl, halo(C₂-₆)alkenyl or halo(C₂-₆)alkynyl. Preferred compounds within this group are those wherein R₁₀ is halo(C₁-₆)alkyl and R₉ is hydrogen or a halogen, particularly where R₁₀ is CF₃ and R₉ is hydrogen, chlorine or fluorine. More preferred compounds within this group are those wherein R₃ is hydrogen and R4 is a halogen or wherein R₃ is halo(C₁-₆)alkyl and R₄ is hydrogen, particularly compounds wherein R₃ is H or CF3 and R₄ is chlorine or hydrogen. Most preferred compounds within this group are those wherein R₃ is CF₃, R₄ and R₁₀ are hydrogen and R₉ is a halogen, particularly chlorine. In these embodiments R₂, R₅, R₆, R_{7,} R₈ and R₁₁ are preferably hydrogen and R₁₈ and R₁₉ are preferably hydrogen, methyl, ethyl or together form a cyclopropyl ring. More preferably R₁₈ and R₁₉ are both methyl.

Preferred compounds within this group are:
4-[m-(Trifluoromethyl)benzyl]-6-[(α,α,α,-trifluoro-m-tolyl)oxy]pyrimidine;
4-(p-Chloro-α,α,-dimethylbenzyl)-6-[(α,α,α,4-tetrafluoro-m-tolyl)oxy]pyrimidine;
4-(p-Chloro-α,α,-dimethylbenzyl)-6-[(α,α,α,4-trifluoro-m-tolyl)oxy]pyrimidine;
4-[1-(p-Chlorophenyl)cyclopropyl]-6-[(α,α,α,-trifluoro-m-tolyl)oxy]pyrimidine;
4-[1-(p-Chlorophenyl)cyclopropyl]-6-[(α,α,α,4-tetrafluoro-m-tolyl)oxy]pyrimidine;
6-[(α,α,α,4-Tetrafluoro-m-tolyl)oxy]-4-pyrimidinyl α,α,α,4-trifluoro-m-tolyl ketone;
4-[α,α-Dimethyl-m-(trifluoro-methyl)benzyl]-6-[(α,α,α,-trifluoro-m-tolyl)oxy]pyrimidine; and
4-[α,α-Dimethyl-m-(trifluoro-methyl)benzyl]-6-[(α,α,α,4-tetrafluoro-m-tolyl)oxy]pyrimidine.

A further preferred group of compounds are those wherein X₁ is C=NOR₂₀ and X₂ is CH₂. Preferred embodiments within this group are those wherein R₃, R₄ and R₉ are independently selected from halo(C₁-₆)alkyl, halo(C₂-₆)alkenyl or halo(C₂-₆)alkynyl. Preferred embodiments of the invention within this group are compounds wherein R₃ and R₁₀ are each hydrogen and at least one of R3 and R₁₀ is halo(C₁-₆)alkyl. More preferred compounds are those wherein R₄ and R₉ are both halo(C₁-₆)alkyl, particularly compounds wherein R₃ and R₁₀ are both CF₃. In these embodiments R₂, R₅, R₆, R₇, R₈ and R₁₁ are preferably hydrogen and R₂₀ is preferably methyl,

A preferred compound within this group is 6-[m-(trifluoromethyl)benzyll-4-pyrimidinyl α,α,α-trifluoro-m-tolyl-ketone, O-methyloxime, (E)- and (Z)-.

A further preferred group of compounds are those compounds wherein one of X₁ and X₂ is NR₁₄ and the other is S or those wherein R₁ is (C₁₋₆)alkylthio, halo(C₁₋₆)-alkylthio, (C₁₋₆)alkylsulfinyl, halo (C₁₋₆)alkylsulfinyl, (C₁₋₆)alkylsulfoxyl, halo (C₁₋₆)alkyl-sulfoxyl or NR₁₂R₁₃; and one of X₁ and X₂ is NR₁₄ and the other is O.

When one of X₁ and X₂ is O and the other is NR₁₄ or NR₁₅ (as defined above) and R₁ is hydrogen, then preferably at least one of R₂, R₃, R₄, R₅, R₆, R₇, R₈, R₉ and R₁₁ is cyano, nitro, (C₁-₆)alkoxy(C₁-₆)alkyl, halo(C₁-₆)alkoxy (C₁-₆)alkyl, halo(C₁-₆)alkylthio, (C₁-₆)alkylsulphinyl, halo(C₁-₆)alkylsulphinyl, (C₁-₆)alkylsulphonyl, halo(C₁-₆) alkylsulphonyl, amino, (C₁-₆)alkylamino, di(C₁-₆) alkyl amino, halo(C₁-₆)alkylamino, (C₁-₆)alkylhalo(C₁-₆) alkylamino, dihalo(C₁-₆)alkylamino, (C₁-₆)alkyloxycarbonyl, halo(C₁-₆)alkyloxycarbonyl, (C₂-₆)alkenyl, halo(C₂-₆) alkenyl, (C₂-₆)alkynyl or halo(C₂-₆)alkynyl.

The compounds of the invention are particularly useful as acaricides or insectides. They are especially useful for the control of herbivorous mites, including but not limited to, Tetranychidae such as *Tetranychus urticae*, *Tetranychus pacificus, Tetranychus kanzawai, Panonychus ulmi, Panonychus citri* and *Oligonychus pratensis;* Tarsonemidae such as *Phytonemus pallidus* and *Polyphagotarsonemus latus;* Eriophyidae such as *Aculus schlechtendali*, *Phyllocoptraka oleivora* and *Eriophyes sheldoni;* and Tenuipalpidae such as *Brevipalpus phoenicis.* In particular, the compounds of this invention are especially useful for the control of *Tetranychus urticae*, Panonychus *ulmi, Panonychus citri* and *Brevipalpus phoenicis.*

Plants which are protected from mite infestations by compounds of the present invention include, but are not limited to, citrus plants such as orange, grapefruit, lemon and lime; pome fruits such as apple, pear and kiwi; stone fruits such as avocado, peach, cherry, fig, olive and mango; vine fruits such as grape, strawberry and raspberry; nut crops such as almond, pecan, walnut, pistachio, cashew, filbert, chestnut, macadamia and brazil nut; field crops such as cotton, corn, soybean, wheat, squash and watermelon, ornamental plants such as flowering plants and shrubs; coffee and tea. The compounds of this invention are especially useful for protecting citrus plants and pome fruits from mite infestations.

To provide control of mites, orchard and ornamental plants are generally treated with a liquid, preferably aqueous, dispersion which contains about 1 g/hl to 100 g/hl, preferably 5 g/hl to 20 g/hl of a formula I compounds. In practice the dispersion is generally applied to the orchard or ornamental plant to runoff.

The compounds of this invention are also useful for control of mites in field crops when applied to the crops in sufficient amount to provide a rate of about 50-400 g/ha of active ingredient, more preferably a rate of about 75-250 g/ha of active ingredient.

The term 'foliage' as used herein includes, but is not limited to, the leaves, buds, fruits, stems, twigs, branches and/or flowers of plants. The term 'orchard plant' as used herein includes, but is not limited to, citrus plants, pome fruits, stone fruits, vine fruits and nut crops. The term 'ornamental plant' as used herein includes, but is not limited to flowering plants and shrubs. The term 'field crop' as used herein includes, but is not limited to, vegetables such as squash and watermelon and row crops such as cotton, corn, soybean and wheat.

The invention also relates to compositions comprising a compound of formula I and an agriculturally acceptable carrier. For example, the compounds of this invention may be formulated as emulsifiable concentrates, flowable concentrates or wettable powders which are diluted with water or other suitable polar solvent, generally in situ, and then applied as a dilute spray. They may also be formulated in water dispersible granules, dry compacted granules, granular formulations, dusts, dust concentrates, suspension concentrates, microemulsions and the like. Such formulations or compositions of the present invention include a compound of this invention (or combinations thereof) admixed with one or more agronomically acceptable inert, solid or liquid carriers

Compounds of the invention wherein X₁ is NR₁₄ and X₂ is O may suitably be prepared by reacting a compound of formula II, wherein R₁ is as defined above and the groups Y are the same or different halogens, 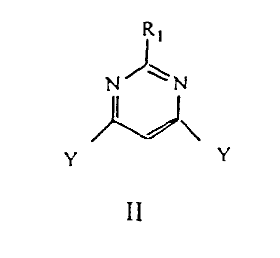 with a compound of formula III 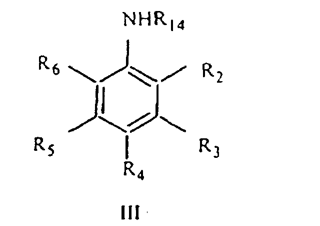 to provide a compound of formula IV 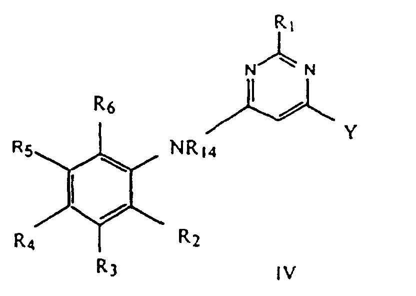 and then reacting the compound of formula IV with a phenol of formula V or a thiophenol of formula V¹ 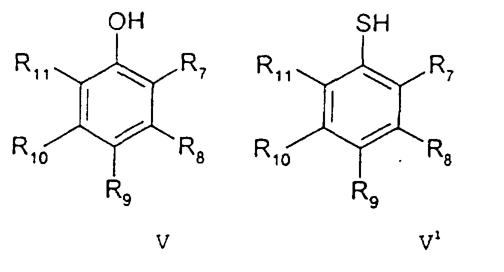 to provide the desired compound of formula I.

Compounds of the invention wherein X₁ is O and X₂ is NR₁₄ may be prepared analogously using the starting materials III^{a} and V^{a}.

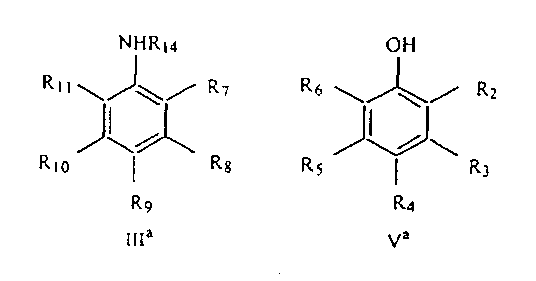

Compounds of the invention wherein X₁ is S and X₂ is NR₁₄ may be prepared analogously using the starting materials III^{a} and V^{1a}

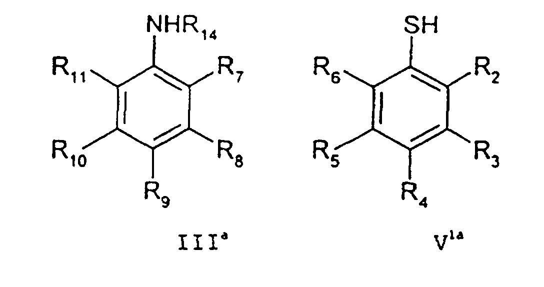

Preparation of compounds of the invention wherein X₁ is CH₂ and X₂ is NR₁₅ may suitably be prepared by:
a) halogenating an acid of formula VI 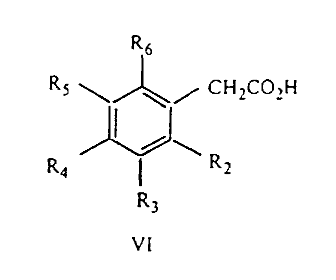 to provide an acid halide of formula VII 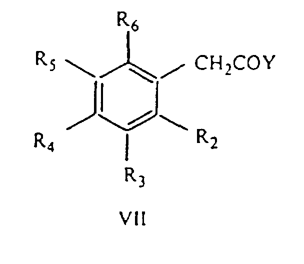 wherein Y is a halogen, e.g. by chlorination, in which case Y is chlorine;
b) alkylating the acid halide of formula VII to provide a compound of formula VIII wherein R₁₈ is an alkyl group e.g. ethyl 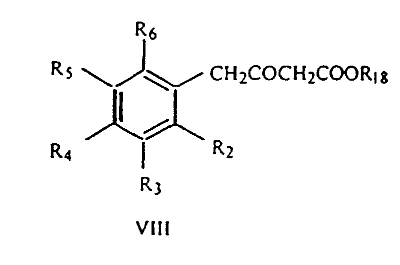
c) converting the compound of formula VIII to provide a compound of formula IX 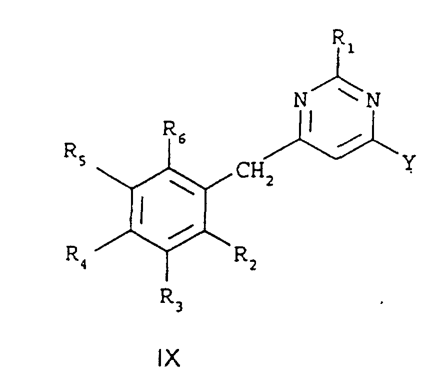 e.g. by first reacting the compound of formula VIII with ammonium acetate in ethanol and then with formamide and potassium tertiary butoxide;
d) halogenating the compound of formula IX to provide a compound of formula X wherein Y is a halogen 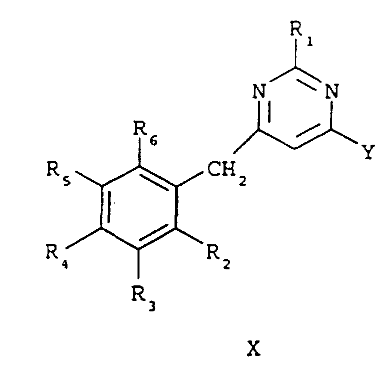 e.g. by chlorination in which case Y is chlorine;
and e) converting the compound of formula X to the desired compound of formula I by reacting it with the appropriate aniline derivative of formula XI

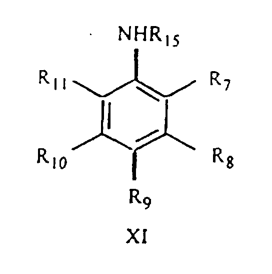

Compounds of formula I wherein X₁ is NR₁₅ and X₂ is CH₂ may be prepared analogously using the starting materials VIa and XI^{a}

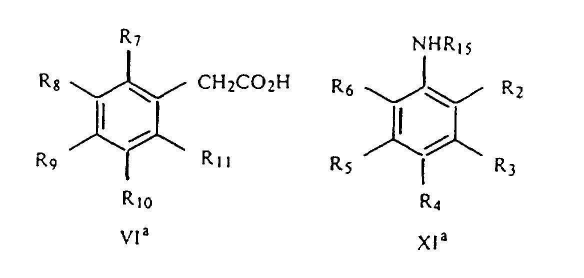

Preparation of compounds of the invention wherein X₁ is CH₂, X₂ is O and R₁ is hydrogen may suitably be prepared by:
a) reacting a compound of formula XII 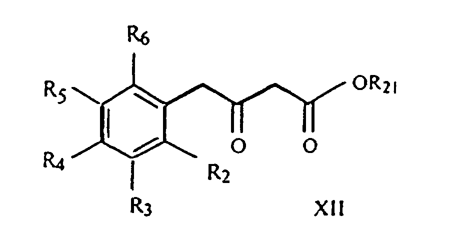 wherein R₂₁ is an alkyl group; with a compound of formula XIII 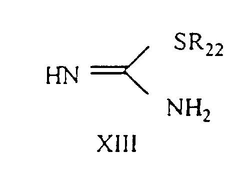 wherein R₂₂ is an alkyl group, to provide a compound of formula XIV, 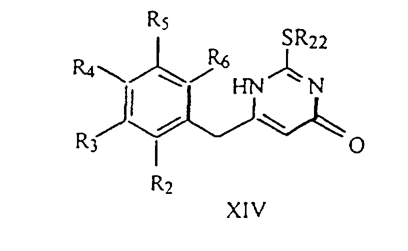 preferably in the presence of a base;
b) halogenating the compound of formula XIV to a compound of formula XV: 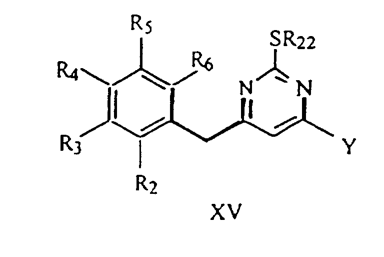 wherein Y is a halogen e.g. chlorine, this may suitable be performed by chlorinating the compound of formula XIV, e.g. using phosphorous oxychloride;
c) reacting the compound of formula XV with a phenol of formula V, as described above, to provide a compound of formula XVII: 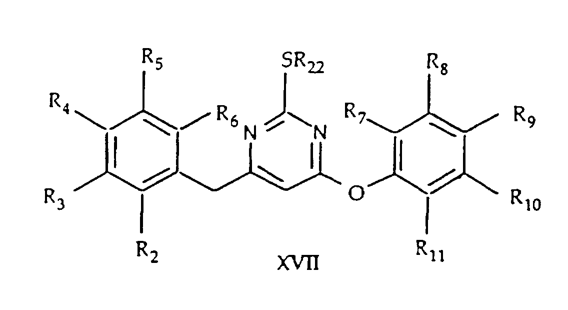
d) oxidising the compound of formula XVII to a compound of formula XVIII: 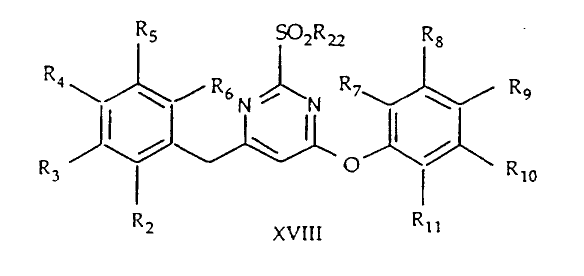 using a suitable oxidising agent, e.g. m-chlorobenzoic acid;
and e) reducing the compound of formula XVIII using a suitable reducing agent, e.g. sodium borohydride, to provide the desired compound of formula I.

Compounds of the invention wherein X₂ is CR₁₈R_{19,} R₁₈ and R₁₉ are both hydrogen, X₁ is O and R₁ is hydrogen may be prepared analogously using the starting materials XII^{a} 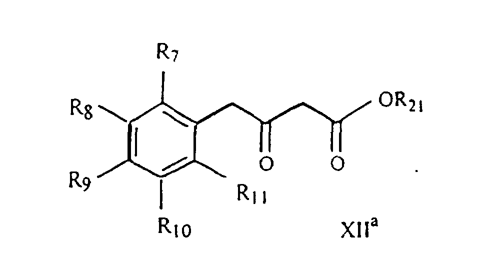 and V^{a} described above.

Compounds of the invention wherein X₁ is CH₂, X₂ is O, R₁ is NR₁₂R₁₃ and R₁₂ and R₁₃ are as defined above may be prepared by reacting the compound of formula XVIII, with an amine of formula XIX:

NHR₁₂R₁₃ XIX

e.g. using diethylamine to introduce a diethyl amino group.

Similarly, compounds of the invention wherein X₂ is CH₂, X₁ is O, R₁ is NR₁₂R₁₃, and R₁₂ and R₁₃ are as defined above, may be prepared analogously by reacting the compound of formula XVIII^{a} 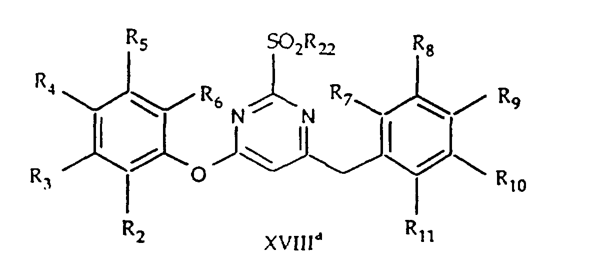 with an amine of formula XIX.

Compounds of the invention wherein X₁ is CR₁₈R₁₉, X₂ is O and R₁₈ and R₁₉ are the same and are selected from (C₁₋₆)alkyl, (C₂₋₆)alkenyl and (C₂₋₆)alkynyl which are all optionally substituted by one or more halogens which are the same or different may be prepared by:
a) reacting a compound of formula XX 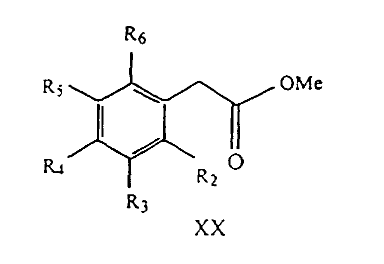 with at least 2 equivalents of an iodide of formula XXI, R₁₈I, preferably in the presence of a base, to provide a compound of formula XXII 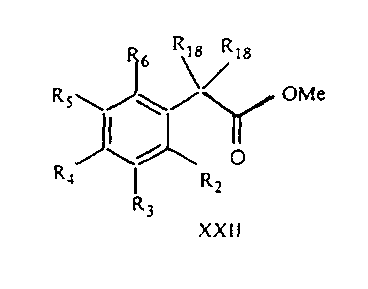
b) saponifying the compound of formula XXII to provide a compound of formula XXIII 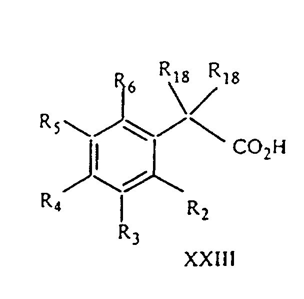
c) converting the compound of formula XXIII to a compound of formula XXIV 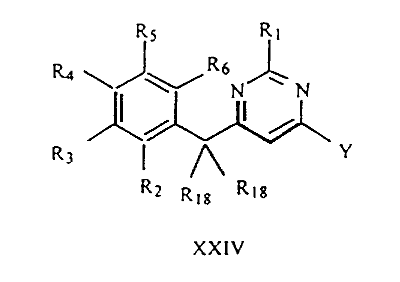 wherein Y is a halogen, and d) reacting the compound of formula XXIV with a phenol of formula V, as described above, to provide the desired compound of the invention.

Compounds of the invention wherein X₁ is O, X₂ is CR₁₈R₁₉, and R₁₈ and R₁₉ are the same and are selected from (C₁-₆)alkyl, (C₂-₆)alkenyl and (C₂-₆)alkynyl which are all optionally substituted by one or more halogens which are the same or different may be prepared analogously.

Compounds wherein R₁₈ and R₁₉ are identical may be prepared using at least 2 equivalents of the same iodide whereas compounds wherein R₁₈ and R₁₉ are different to one another may be prepared by the sequential use of no greater than one equivalent of each of two different alkyl halides. Spiro compounds may be prepared from appropriate alkyldihalides.

Compounds of the invention wherein X₁ is CO and X₂ is O may be prepared from the corresponding compound wherein X₁ is CH₂, e.g. by air oxidation. Similarly compounds of the invention wherein X₁ is O and X₂ is CO may suitably be prepared by converting the corresponding compound wherein X₂ is CH₂.

Compounds of the invention wherein X₁ is C=NOR₂₀ and X₂ is CH₂ may be prepared by:
a) converting the compound of formula X^{a} 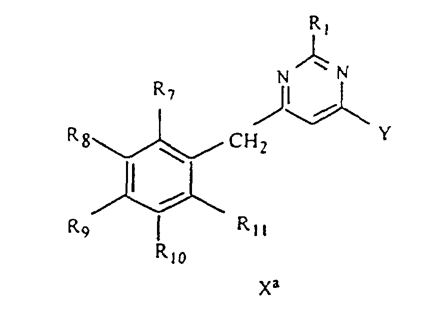 to a compound of formula XXVIII 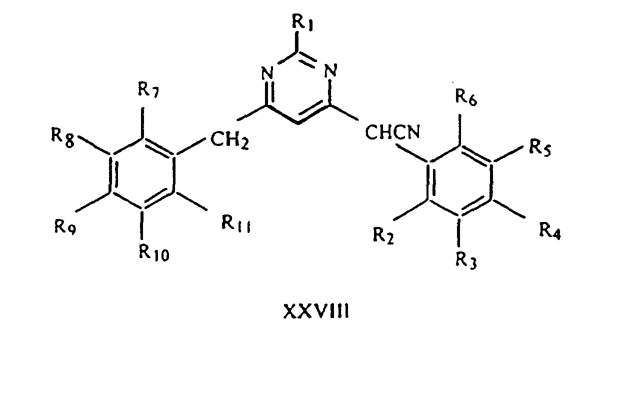
b) treating the compound of formula XXVIII with aqueous base to provide a compound of formula XXIX 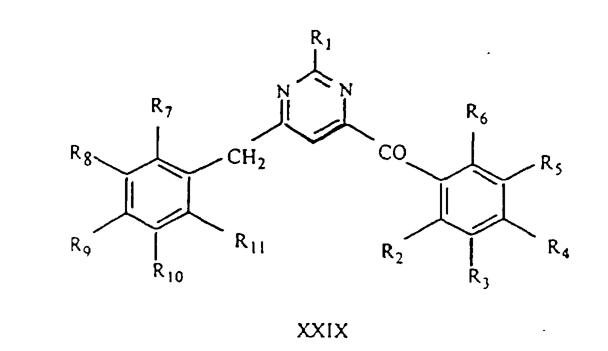
and c) converting the compound of formula XXIX into the corresponding oxime.

Compounds of the invention wherein X₂ is C=NOR₂₀ and X₁ is CH₂ may be similarly prepared by using compounds of formula X and V^{a}, as described above, as starting materials.

Accordingly, the present invention also provides a process for the preparation of a compound of formula I which comprises the following steps:
i) reacting a compound of formula IV 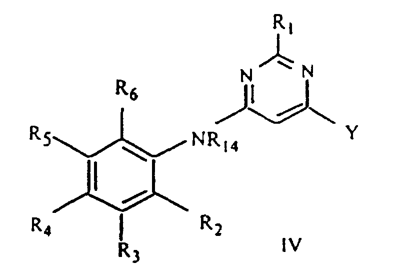 with a phenol of formula V 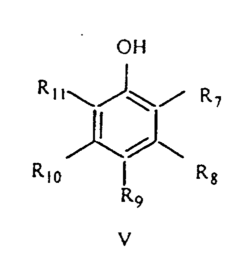 to provide a compound of formula I wherein X₁ is NR₁₄ and X₂ is O;
ii) reacting a compound of formula IV^{a} 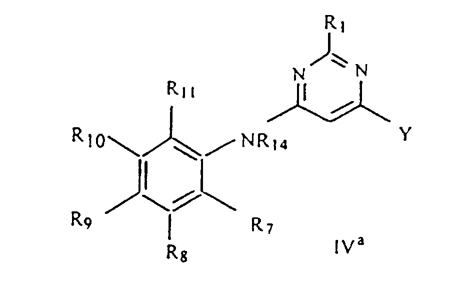 with a phenol of formula V^{a} 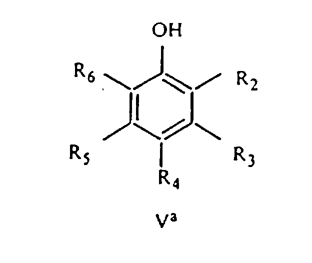 to provide a compound of formula I wherein X₁ is O and X₂ is NR₁₄;
iii) reacting a compound of formula X 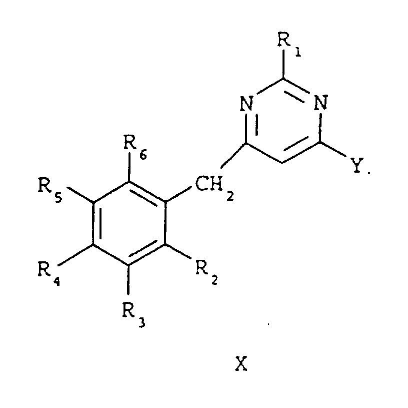 wherein Y is a halogen, with the appropriate aniline derivative of formula XI 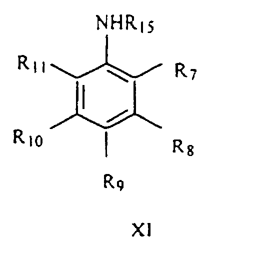 to provide a compound of formula I wherein X₁ is CH₂ and X₂ is NR₁₅;
iv) reacting a compound of formula X^{a} 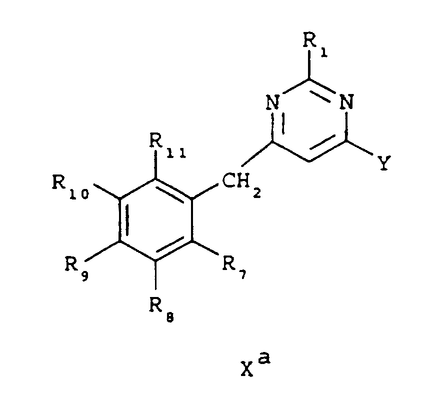 wherein Y is a halogen, with the appropriate aniline derivative of formula XI^{a} 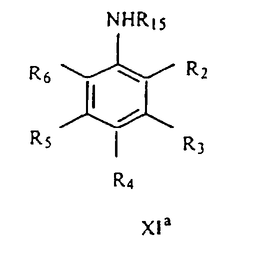 to provide a compound of formula I wherein X₁ is NR₁₅ and X₂ is CH₂;
v) reducing a compound of formula XVIII 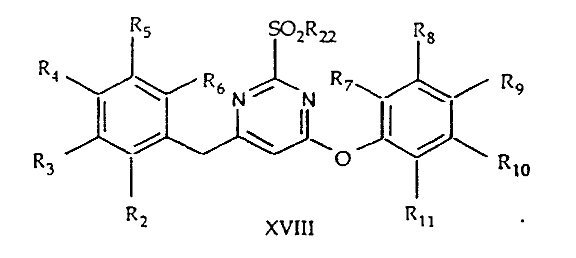 to provide a compound of formula I wherein X₁ is CH₂, X₂ is O and R₁ is hydrogen;
vi) reducing a compound of formula XVIII^{a} 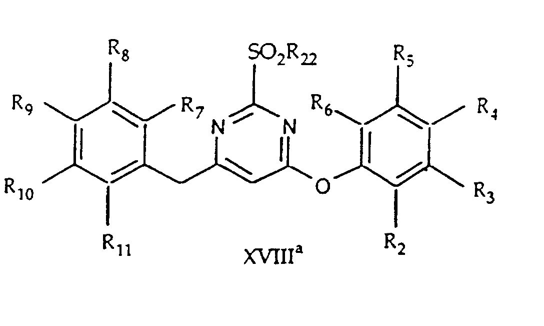 to provide a compound of formula I wherein X₁ is O, CH₂, X is O and R₁ is hydrogen;
vii) reacting a compound of formula XVIII with an amine of formula XIX:

   NHR₁₂R₁₃ XIX

   to provide a compound of formula I wherein X₁ is CH₂, X₂ is O and R₁ is NR₁₂R₁₃;
viii) reacting a compound of formula XVIII^{a} with an amine of formula XIX, as described above, to provide a compound of formula I wherein X₁ is O, X₂ is CH₂ and R₁ is NR₁₂R₁₃;
ix) reacting a compound of formula XXIV 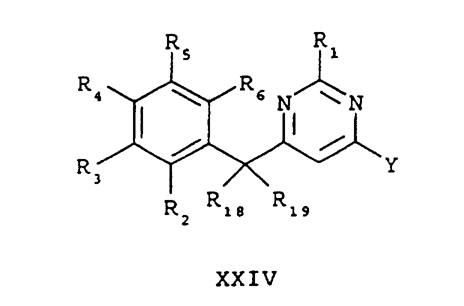 with a phenol of formula XVI 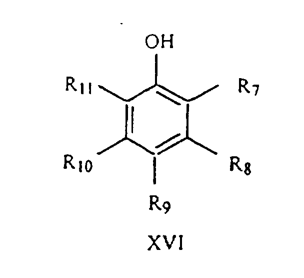 to provide a compound of formula I wherein X₁ is CR₁₈R_{19,} X₂ is O and R₁₈ and R₁₉ are each selected from (C₁-₆)alkyl, (C₂-₆)alkenyl and (C₂-₆)alkynyl which are all optionally substituted by one or more halogens which are the same or different, or R₁₈ and R₁₉ may together form a carbocyclic ring which is optionally substituted by one or more halogens which are the same or different;
x) reacting a compound of formula XXIV^{a} 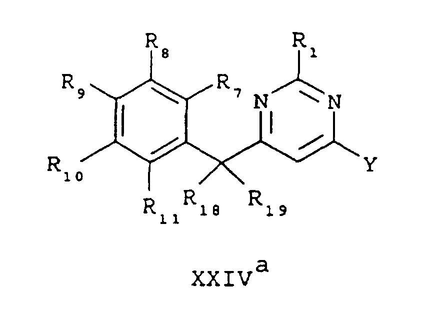 wherein Y is a halogen, with a phenol of formula V^{a} 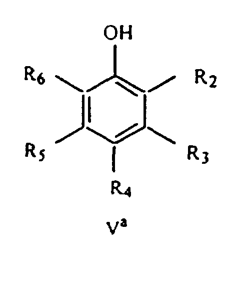 to provide a compound of formula I wherein X₁ is O and X₂ is CR₁₈R_{19,} wherein R₁₈ and R₁₉ are each selected from (C₁-₆)alkyl, (C₂-₆)alkenyl and (C₂-₆)alkynyl which are all optionally substituted by one or more halogens which are the same or different, or R₁₈ and R₁₉ may together form a carbocyclic ring which is optionally substituted by one or more halogens which are the same or different;
xi) converting a compound of formula I wherein X₁ is CR₁₈R₁₉ and X₂ is O to the corresponding compound wherein X₁ is CO;
xii) converting a compound of formula I wherein X₂ is CR₁₈R₁₉ and X₁ is O to the corresponding compound wherein X₂ is CO;
xiii) converting a compound of formula XXIX 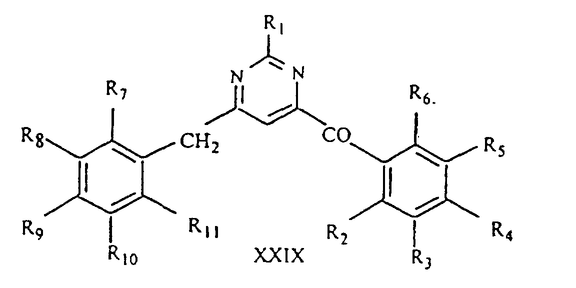 into the corresponding oxime to provide a compound of formula I wherein X₁ is C=NOR₂₀ and X₂ is CH₂;
and xiv) converting a compound of formula XXIX^{a} 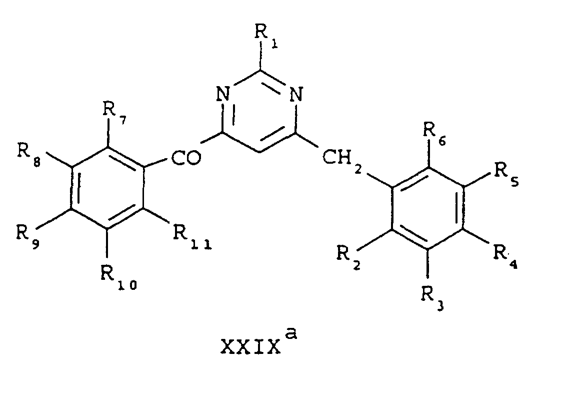 into the corresponding oxime to provide a compound of formula I wherein X₁ is CH₂ and X₂ is C=NOR₂₀.

For a more clear understanding of the present invention, the following examples are presented by way of illustration only. The invention is not to be deemed limited thereby.

### EXAMPLE 1

### Preparation of Pyrimidine, 4 -(a, α,α,4-tetrafluoro-m-toluidino)-6-[m-(trifluoromethyl)benzyl] hydrochloride

(3-Trifluoromethylphenyl)acetic acid (25.1 g, 0.12 mol) is added to a solution of dichloromethane (200 ml) and dimethylformamide (1 ml). Thionyl chloride (18.2 g, 0.15 mol) is then added dropwise and the resulting solution is stirred overnight. The solution is then concentrated under reduced pressure to give the crude acid chloride in nearly quantitative yield. The potassium salt of ethyl malonate (41.3 g, 0.24 mmol) is dissolved in acetonitrile (400 ml) and iscooled to 0°C. Triethylamine (23.23g, 0.23 mol) and magnesium chloride(28.8g, 0.30 mol) are then added and the slurry is warmed to 25° C. and is stirred for 2 hours. The slurry then is cooled to 0° C. and the acid chloride is added over 1 hour. The mixture is allowed to warm to room temperature and is stirred overnight. The solvent is removed under reduced pressure and the resulting solid is suspended in toluene (200 ml) and is cooled to 0° C. Ice cold 4N hydrochloric acid is then slowly added. The organic phase is separated and washed with 4N hydrochloric acid, water and finally saturated brine. The organic phase is dried and concentrated. The crude product is then purified by distillation to give ethyl(3-trifluoromethylphenyl) acetoacetate (15.4 g).

Ethyl-(3-trifluoromethylphenyl)acetoacetate (15.3 g, 0.056 mol) is added to a solution of ammonium acetate (21.6 g, 0.28 mol) in ethanol (100 ml) and the mixture is refluxed for 3 hours. The solution is concentrated under reduced pressure and then suspended in ethyl acetate. The suspension is washed with water and saturated brine and the organic layer concentrated. The material is then dissolved in dimethylsulfoxide (125 ml) and formamide (20 ml) and potassium tert-butoxide (1.28 g, 0.011 mol) are added. The solution is heated for 18 hours at 100° C. Upon cooling the solution is poured into ice (1000 ml) and glacial acetic acid (20 ml). The resulting solid is collected and air dried. The material is then triturated with diethyl ether and is air dried to give 6-(3-trifluoromethylbenzyl)pyrimidin-4-one (7.20 g).

Under nitrogen atmosphere, 6-(3-trifluoromethylbenzyl)pyrimidin-4-one (7.0 g, 0.027 mol) is dissolved in phosphorus oxychloride (30 ml) and is refluxed for 1.5 hours. After cooling the solution is concentrated under reduced pressure. The solid is then dissolved in ethyl acetate, washed with water, saturated brine, dried and the crude product purified by flash column chromatography to give 6-(3-trifluoromethylbenzyl)-4-chloro-pyrimidine (6.3 g).

6-(3-Trifluoromethylbenzyl)-4-chloro-pyrimidine (1.5 g, 0.0055 mol) and 3-trifluoromethyl-4-fluoroaniline (0.92 g, 0.0055 mol) is heated together in the absence of solvent at 100° C for 1 h. The resulting solid is washed with diethyl ether and air dried to give 4-(α,α,α,4-tetrafluoro-m-toluidino)-6-[m-(trifluoromethyl)benzyl]pyrimidine hydrochloride (2.2 g).

### EXAMPLES 2-16

### Preparation Of Substituted Pyrimidine Compounds

Using essentially the same procedure described hereinabove for Example 1 and employing the appropriate starting materials and reagents, the following compounds wherein X₁ is CH₂ and X₂ is NR15 and R₁, R₂, R₅, R₆, R₇, R₈ and R₁₁ are all hydrogen are prepared.

| Example | R₄ | R₃ | R₉ | R₁₀ | R₁₅ | M.p (C) |
|---|---|---|---|---|---|---|
| 2 | Cl | Cl | F | CF₃ | H | 222-225 |
| 3 | H | CF₃ | Cl | CF₃ | H | 238-241 |
| 4 | H | CF₃ | Cl | CF₃ | H | 228-231 |
| 5 | Cl | CF₃ | F | CF₃ | Me | 96.5-98.5 |
| 6 | Cl | Cl | Cl | CF₃ | Me | 72-75 |
| 7 | Cl | Cl | F | CF₃ | Me | 60-63 |
| 8 | Cl | Cl | Cl | CF₃ | H | 93-95 |
| 9 | Cl | Cl | F | CF₃ | CH(OEt)2 | oil |
| 10 | H | H | Cl | CF₃ | Me | 92-94 |
| 11 | Cl | Cl | H | CF₃ | H | 111-113 |
| 12 | Cl | Cl | H | CF₃ | Me | 62-64 |
| 13 | F | F | F | CF₃ | H | 45-47 |
| 14 | F | F | F | CF₃ | Me | 104-107 |
| 15 | F | F | Cl | CF₃ | H | 45-50 |
| 16 | F | F | Cl | CF₃ | Me | 91-94 |

### EXAMPLE 17

### Insecticidal And Acaricidal Evaluation Of Test Compounds

The activity of the compounds prepared is determined by preparing stock solutions of test samples at 4000 ppm in acetone/water and diluted appropriately for the following assays:

### Southern Army Worm (Spodoptera eridania, SAW)

Excised lima bean leaves are dipped into the test solution and held upright for 10 minutes to allow complete evaporation of the solvent. Four leaves are placed in a test cup (8 oz, paper) and 3 sheets of filter paper added. Each cup is treated with 5 second instar S. eridania larvae and the cup capped. Test cups are maintained at 27-28° C and 50% humidity for 5 days. Mortality is then observed and rated according to the scale below.

### Twospotted Spider Mite (Tetranychus urticae, TSM)

Lima bean plants grown to the primary leaf stage with leaves approximately 7 cm in length are infested with mites by placing a heavily infested leaf on each leaf of the test plant. Mites are allowed to transfer for at least two hours. The infested leaves of the intact plant are dipped into the test solution to provide complete coverage of the leaf surfaces. Plants are maintained under GRO-LUX lights (24-hour photoperiod) at 27-28° C for 5 days. Mortality is observed of treat mites and surviving population and rated according to the scale below.

### Western Corn Rootworm (Diabrotica virgifera virgifera, WCR)

Millet seeds (*Panicum spp*.) are soaked in water one day prior to testing to initiate seed germination. Test solutions are added to 4 fl.oz. specimen cups and the acetone is allowed to evaporate. Dry soil (30 g / cup) is added to each cup and the water/test material solution is allowed to disperse through the soil for a minimum of 1 hour. Presoaked millet seed (1 cc.) is added to each cup and the cup is shaken vigorously to incorporate the test material and seed evenly throughout the soil. Each cup is then infested with 10 second instar WCR larvae and re-capped. Five small holes are inserted in each cup lid for ventilation. Test cups are maintained at 27-28° C and 50% humidity for 5 days. Mortality is then observed and rated according to the scale below.

### Insecticide Diet Assay (Miniscreen)

Targets: Southern Armyworm (*Spodoptera eridania*, SAW)and Southern Corn Rootworm *(Diabrotica undecimpunctata howardi*, SCR).

Insect diet (Southland for SAW, and Bioserve #9800 for SCR) is poured into 128 well bioassay trays. Test compounds, rendered in 96-well deepwell microtiter plates, are reconstituted to the appropriate test concentration (3000 ppm for SAW and 1000 ppm for SCR) using 100% acetone as the solvent. A total of four diet wells (two reps for each species) are each treated with 50ml of test solution. The acetone is allowed to evaporate leaving the dried compound covering the surface of the diet well. Eggs of each test target are suspended in a 0.2 % agar solution at a density of 10-20 eggs per 20 ml. A total of 20ml of each egg suspension is pipetted onto the surface of each treated well on the appropriate diet. The agar is allowed to dry off leaving the eggs sitting on the surface of the contaminated diet. The trays are covered with vented, resealable lids and incubated at 25° C for seven days. Mortality is then observed according to the scale below.

The results are rated according to the following table :
0 = no effect 5 = 56-65%
1 = 10-25% 6 = 66-75%
2 = 26-35% 7 = 76-85%
3 = 36-45% 8 = 86-99%
4 = 46-55% 9 = 100%
The results are shown in the following table:

| | TSM twospotted mite | | | SAW southern army worm | | | | |
|---|---|---|---|---|---|---|---|---|
| Example | 300 (ppm) | 100 (ppm) | 10 (ppm) | 3000 (ppm) | 1000 (ppm) | 300 (ppm) | 100 (ppm) | 10 (ppm) |
| 1 | 9 | 8 | 0 | | | 9 | 7 | 0 |
| 2 | 8 | 0 | 0 | | 0 | 0 | | |
| 3 | 6 | | | | | 4 | | |
| 4 | 0 | | | | | 1 | | |
| 5 | 2 | | | | | 4 | | |
| 6 | 8 | 7 | 3 | | 9 | 1 | | |
| 7 | 9 | 5 | 0 | | | 0 | | |
| 8 | 8 | 0 | 0 | | | 0 | | |
| 9 | 9 | 8 | 2 | | | 0 | | |
| 10 | 9 | 0 | 0 | | | 1 | | |
| 11 | 0 | | | | | 1 | | |
| 12 | 0 | | | 9 | | 0 | | |
| 13 | 9 | 9 | 0 | | | 2 | | |
| 14 | 9 | 9 | 7 | | | 9 | 4 | 0 |
| 15 | 8 | 6 | 0 | | | 7 | 0 | 0 |
| 16 | 9 | 9 | 0 | | | 3 | | |

### EXAMPLE 18

### Preparation And Evaluation Of 6-[m-(Trifluoromethyl) benzyl] -4-pyrimidinyl-α,α,α-trifluoro-m-tolyl-ketone, O-methyloxime (E)- and (Z)-

3-Trifluoromethylphenylacetonitrile (2.04g, 11 mmol) is added to THF (100 ml) under nitrogen and the reaction is chilled to -65° C. t-Butyl lithium (1.7N, 13 ml, 22 mmol) is then added slowly and after a further 5 minutes 4-chloro-6-(3-trifluoromethylbenzyl pyrimidine (3.0 g, 11 mmol) in THF (50 ml) is slowly added. The reaction is stirred at -65° C for 1 hour and then at room temperature for 18 hours before being poured into ethyl acetate and 10% hydrochloric acid. The product is extracted into ethyl acetate, is washed with 10% hydrochloric acid and the combined organic layers dried over sodium sulphate. The organic solution is filtered and concentrated under vacuum to give 4.8g brown solid which is triturated with methylene chloride to give 4-(3-trifluorophenylacetonitrile)-6-(3-trifluoromethyl-benzyl)pyrimidine (1.8 g) as a yellow powder. The filtrate is chromatographed to obtain 0.45 g additional material. ¹H NMR CDCl₃ 4.18 (2H,s) 5.25 (1H,s) 7.3 (1H,d) 7.44-7.67 (8H,m) 9.13 (1H,d).

4-(3-Trifluorophenylacetonitrile)-6-(3-trifluoromethylbenzyl)pyrimidine (3.28 g, 7.8 mmol) is added to toluene (250 ml), sodium hydroxide (3.1 g, 78.0mmoles) in water (150 ml) and tetrabutyl ammonium chloride (0.21 g, 0.78mmol). The reaction is stirred at room temperature for 18 h at which time tetrabutyl ammonium chloride (0.4 g, 1.44mmol) is added and the reaction mixture is stirred for 24 hours at room temperature. The product is then extracted into ethyl acetate, washed with brine, dried over sodium sulphate, filtered and concentrated to give a brown liquid. The crude product is chromatographed to give 1.4 g of 6-[m-(Trifluoromethyl)benzyl]-4-pyrimidinyl alpha,alpha,alpha-trifluoro-m-tolyl ketone as a yellow oil. ¹H NMR CDCl₃ 4.30 (2H, s) 7.50-8.42 (8H, m) 7.8 (1H, d) 9.32 (1H, d)

6-[m-(Trifluoromethyl)benzyl)-4-pyrimidinyl alpha, alpha,alpha-trifluoro-m-tolyl ketone (0.45g, 1.1mmol), ethanol (50 ml) and O-methylhydroxylamine hydrochloride (0.45g, 5.3mmol) are combined and refluxed for 6 hours. The solvent is removed under vacuum, the residue is taken up in ethyl acetate and is washed with water, brine, dried over magnesium sulfate and the solvent is removed under vacuum to yield 0.30 g yellow oil of the syn and anti isomers of the title compound. ¹H NMR CDCl₃ 3.99, 4.06 (3H, s) 4.21, 4.27 (2H, s) 7.49-7.80 (9H, m) 9.10, 9.30 (1H, d)

The activity of this compound is measured as described above and the results are shown below:

| | TSM | | | SAW | | | |
|---|---|---|---|---|---|---|---|
| Example | 300 (ppm) | 100 (ppm) | 10 (ppm) | 1000 (ppm) | 300 (ppm) | 100 (ppm) | 10 (ppm) |
| 18 | 7 | 5 | 0 | | 0 | | |

### EXAMPLE 19

### Preparation Of 4-(α,α,α-Trifluoro-N-methyl-m-toluidino)-6-[(α,α,α-trifluoro-m-tolyl)oxy]pyrimidine

4,6-Dichloropyrimidine (1.55 g, 0.01mol) and N-methyl-3-trifluoromethylaniline (2.0 g, 0.11mol) are combined and heated to 126° C under N₂ for 1.5 hours. The resulting oil is taken up in ethyl acetate, is washed with 10% sodium carbonate and dried over sodium sulphate. The organic phase is concentrated under reduced pressure to yield a yellow oil which is purified by column chromatography to give 4-chloro-6-(N-methyl-3-trifluoromethylanilino)-pyrimidine (1.78 g) as a white solid.

4-Chloro-6-(N-methyl-3-trifluoromethylanilino)-pyrimidine (0.30 g, 0.001 mol.) and sodium carbonate (0.28g, 0.003 mol.) in DMF are stirred for 10 minutes. m-Trifluoro-methylphenol (0.16g, 0.001mol) is added and the mixture is heated to 60° C for 12 hours. The mixture is then cooled and poured into water. The product is extracted into ethyl acetate, washed with 10% sodium hydroxide, 10% hydrochloric acid, and 5% potassium carbonate, dried over Na₂SO₄, filtered, and is concentrated under reduced pressure. 6-(N-methyl-3-trifluoromethylanilino)-4-(3-trifluoromethylphenoxy)pyrimidine (0.16g) obtained by chromatography as a white solid. M.p. 118-119° C.

### EXAMPLES 20-29

### Preparation And Evaluation Of Substituted Pyrimidine Compounds

Using essentially the same procedures described in Example 19 hereinabove, the following compounds in which X₁ is NR₁₄, X2 is O and R₁, R₂, R₅, R₆, R₇, R₈ and R₁₁ are all hydrogen are prepared.

| Example | R₄ | R₃ | R₉ | R₁₀ | R14 | M.p (C) |
|---|---|---|---|---|---|---|
| 20 | H | CF₃ | F | CF₃ | Me | 119-120 |
| 21 | H | CF₃ | Cl | Cl | Me | 70-71 |
| 22 | H | CF₃ | Cl | CF₃ | Me | 101-102 |
| 23 | F | CF₃ | H | CF₃ | Me | |
| 24 | F | CF₃ | Cl | CF₃ | Me | |
| 25 | Cl | CF₃ | F | CF₃ | Me | |
| 26 | Cl | Cl | H | CF₃ | Me | |
| 27 | Cl | Cl | F | CF₃ | Me | |
| 28 | Cl | Cl | Cl | Cl | Me | |
| 29 | F | CF₃ | F | CF₃ | Me | |

The NMR characteristics of these compounds are shown below:
- Example 23: CDCl₃:: ¹H NMR: 8.36(1H,s), 7.64-7.28(7H,m), 5.87(1H,s),3.48(3H,s)
- Example 24: CDCl₃:: ¹H NMR: 8.34(1H,s), 7.50-7.00(6H,m), 5.90(1H,s), 3.49(3H,s)
- Example 25: CDCl₃ :: ¹H NMR: 8.34(1H,s), 7.64-7.19(6H,m), 5.97(1H, s), 3.50(3H,s)
- Example 26: CDCl₃:: ¹H NMR: 8.36(1H,s), 7,52-7,12(6H,m), 5.95(1H, s), 3.46(3H, s)
- Example 27: CDCl₃:: ¹H NMR: 8.34 (1H,s), 7.54-7.15(6H,m), 5.98(1H,s),3.48(3H,s)
- Example 28: CDCl₃:: ¹H NMR: 8.35(1H,s), 7.53-6.95(6H,m), 5.94(1H,s), 3.46(3H,s)
- Example 29: CDCl₃:: ¹H NMR:3.49 (3H, s) 5.85 (1H,d) 7.20-7.75(6H,m) 8.35 (1H,d)

The activity of these compounds is shown below:

| | TSM | | | | SAW | | | | WCR |
|---|---|---|---|---|---|---|---|---|---|
| Example | 300 (ppm) | 100 (ppm) | 10 (ppm) | 1 (ppm) | 1000 (ppm) | 300 (ppm) | 100 (ppm) | 10 (ppm) | 50 (ppm) |
| 19 | 0 | | | | | 0 | | | 2 |
| 20 | 0 | | | | | 6 | 3 | 1 | |
| 21 | 9 | 0 | 0 | | | 0 | 0 | | |
| 22 | 0 | | | | | 3 | | | |
| 23 | 9 | 9 | 5 | 0 | | 9 | 7 | 0 | |
| 24 | 9 | 9 | 9 | 0 | | 9 | 4 | 0 | |
| 25 | 9 | 9 | 9 | 0 | | 4 | 5 | 0 | |
| 26 | 9 | | | | 9 | | | | |
| 27 | 9 | | | | 9 | | | | |
| 28 | 1 | | | | 3 | | | | |
| 29 | 9 | 9 | 9 | 6 | 9 | 9 | 9 | 1 | |

### EXAMPLE 30

### Preparation Of 4-[m-(Trifluoromethyl)benzyl]-6-[(α,α,α-trifluoro-m-tolyl)oxy]pyrimidine

Ethyl-(3-trifluoromethylphenyl)acetoacetate (15.8 g, 57.6mmol) is combined with ethanol (60 ml), water (60ml) and 2-ethyl-2-thiopseudourea hydrobromide (12.8 g, 69.lmmol). Potassium carbonate (9.5 g, 69.1mmol) is added portionwise at room temperature over 1 minute under nitrogen and the reaction is stirred 18 hours. The reaction is added to 200 ml water and acetic acid (4.2 g, 69.lmmol) and stirred for 5 minutes. The resulting white precipitate is filtered, taken up in ethyl acetate, washed with water, brine, and dried over magnesium sulfate. The solvent is removed under vacuum and the resulting solid is triturated with 240 ml heptane to afford 10.1g of 2-thioethyl-6-(3-trifluoromethylbenzyl)-4-(3H)-pyrimidin-one as a white flocculant solid. Mp 119-124° C.

2-Thioethyl-6-(3-trifluoromethylbenzyl)-4-(3H)-pyrimidin-one (5.8 g, 18.5mmol) is suspended in phosphorous oxychloride (40 ml). N,N-dimethylaniline (2.24 g, 18.5mmol) is added and the reaction is stirred for 2 hours at room temperature. The unchanged phosphorous oxychloride is removed under vacuum and the resulting red liquid (6.3 g) is directly in the next step.

4-Chloro-2-thioethyl-6-(3-trifluoromethylbenzyl)-pyrimidine (4.0g, 18.5mmol) is dissolved in DMF (40 ml). 3-trifluoromethylphenol (3.6 g, 22.2mmol) and potassium carbonate (3.02 g, 22.2mmol) are added and the reaction is heated under nitrogen at 50°C for 19 hours and 100° C for 4 hours. The reaction is partitioned between ethyl acetate and water. The organic is washed with water, brine,dried over magnesium sulfate and concentrated to give a brown oil which is filtered through a silica plug (ethyl acetate eluent). Heptane (20 ml) is added to the resulting oil and it is allowed to stand at 5°C overnight. The resulting yellow solid is filtered to give 4.0g of the 2-thioethyl-4-(3-trifluoromethylbenzyl)-6-(3-trifluoromethyl-phenoxy)-pyrimidine. ¹H NMR CDCl₃ 1.14 (3H, t) 2.83 (2H,q) 4.06 (2H, s) 6.34 (1H, s) 7.3-7.6 (8H, m)

2-Thioethyl-4-(3-trifluoromethylbenzyl)-6-(3-trifluoro-methylphenoxy)-pyrimidine (4.0 g, 8.7mmol) is dissolved in methylene chloride (50ml). *m*-chloroper-benzoic acid (60%, 5.0 g, 17.5mmol) is added portionwise over 2 minutes and the reaction is stirred for one hour at room temperature. The solvent is removed under vacuum and the residue is taken up in ethyl acetate and is washed with 5% sodium metabisulfite, saturated sodium bicarbonate, brine and dried over magnesium sulfate. The solvent is removed under vacuum to give 4.0g of oil which is chromatographed yielding 2.8 g 2-(ethylsulfonyl)-4-[m-(trifluoromethyl)benzyl] -6- [(α,α,α-trifluoro-m-tolyl)oxy]pyrimidine as a clear viscous oil. ¹H NMR CDCl₃ 1.27 (3H, t) 3.32 (2H,q) 4.29 (2H, s) 6.81 (1H, s) 7.3-7.6 (8H)

2- (Ethylsulfonyl) -4- [m- (trifluoromethyl)benzyl] -6-[(α,α,α-trifluoro-m-tolyl)oxy]pyrimidine (1.17g, 2.4mmol) is added to chloroform (10 ml) and ethanol (10 ml). sodium borohydride (0.3 g, 7.9mmol) is added portionwise over 15 minutes and the reaction is stirred at room temperature for 80 mins. The mixture is taken up in ethyl acetate and is washed with water, brine and dried over magnesium sulfate. The solvent is evaporated to give 1.05g of a cloudy oil which is chromatographed to yield 0.89g 4-[m-(trifluoromethyl)benzyl]-6-[(α,α,α-trifluoro-m-tolyl)oxy]pyrimidine as a water white oil. ¹H NMR CDCl₃ 4.17 (2H, s) 6.75 (1H,s) 7.3-7.6 (8H, m) 8.71 (1H, s)

### EXAMPLE 31

### Preparation Of 2-(Dimethylamino)-4-[m-(trifluoromethyl)benzyl]-6-[(alpha,alpha,alpha-trifluoro-m-tolyl)oxy]pyrimidine

2-(Ethylsulfonyl)-4-[m-(trifluoromethyl)benzyl] -6-[(α,α,α-trifluoro-m-tolyl)oxy]pyrimidine (0.40g, 0.82 mmol) is added to THF (20 ml). Dimethylamine hydrochloride (0.11 g, 13.4 mmol) and triethylamine (0.17 g, 16.8 mmol) is added and the reaction stirred at room temperature for 18 days. Ethyl acetate is added to the reaction and the organic phase is washed with water, brine and dried over magnesium sulfate. The solvent is removed under vacuum and the residue is chromatographed to afford 0.40g 2- (Dimethylamino)-4- [m-(trifluoromethyl)-benzyl] -6-[(α,α,α-trifluoro-m-tolyl)oxy]pyrimidine as a yellow oil. ¹H NMR CDCl₃ 3.02 (6H, b) 3.92 (2H, s) 5.87 (1H, s) 7.3-7.65 (8H, m).

### EXAMPLE 32

### Preparation Of 4-[(α,α-Dimethyl-m-(trifluoromethyl) benzyl] -6-[(α,α,α,4-tetrafluoro-m-tolvl)oxy]pyrimidine

(3-Trifluoromethyl phenyl)acetic acid methyl ester (30g, 0.138mol), is added to THF (200 ml) under nitrogen. The reaction is cooled to -70° C and (35.3g, 0.315 mol) potassium *tert*-butoxide in THF (300 ml) is added slowly. The reaction is stirred at -70° C for 10 minutes and methyl iodide (44.7 g, 0.315 mol) is added slowly. The reaction is stirred for 3 hours while allowing the temperature to rise to -30° C and further stirred at room temperature for 18 h. The reaction is quenched with 10% hydrochloric acid and the product extracted into ethyl acetate. The organic phase is washed with water, saturated sodium meta bisulfite and dried over magnesium sulfate. The solvent is removed under vacuum to yield 29.1 g α,α-dimethyl-(3-trifluoro-methylphenyl)acetic acid methyl ester as a yellow liquid. ¹H NMR CDCl₃ 1.62 (6H, s) 3.67 (3H, s) 7.4-7.6 (4H,m)

α,α-Dimethyl-(3-trifluoromethylphenyl)acetic acid methyl ester (24.6g, 0.1 mol), acetic acid (150 ml), water (75ml) and sulfuric acid (36g, 0.36 mol) are combined. The reaction is refluxed for 3 hours and stirred at room temperature for 3 days. The reaction is concentrated to half its original volume and added to ethyl acetate and washed with water, dried over magnesium sulfate and concentrated under reduced pressure to give 16.6 g α,α-dimethyl-(3-trifluoromethylphenyl)acetic acid as a white solid. ¹H NMR CDCl₃ 1.63 (6H,s) 7.45-7.65 (4H, m)

The α,α-dimethyl-(3-trifluoromethylphenyl)acetic acid is converted to 6-chloro 4-(α,α-dimethyl-3-trifluoromethylbenzyl)-pyrimidine as described above in Example 1.

6-Chloro 4-(α,α-dimethyl-3-trifluoromethylbenzyl)-pyrimidine (10.45 g, 1.5 mmol), 4-fluoro-3-trifluoromethylphenol (0.30g, 1.65 mmol) and potassium carbonate (0.30g, 2.2 mmol) are combined in 15 ml of dimethyl formamide and heated to 70° C for 18 hours. The reaction mixture is added to ethyl acetate, washed with water, 2.5% sodium hydroxide, brine and dried over magnesium sulfate. The solvent is removed under vacuum to afford 1.02g of a brown oil which is chromatographed to afford 0.93g of the title compound as a yellow oil. ¹H NMR CDCl₃ 1.79 (6H, s) 6.78 (1H, d) 7.2-7.6 (7H, m) 8.71 (1H, d)

### EXAMPLES 33-39

### Preparation Of Substituted Pyrimidine Compounds

Using essentially the same procedure described hereinabove for Example 32 and employing the appropriate starting materials and reagents, the following compounds in which X₁ is CR₁₈R₁₉, X₂ is O and R₁, R₂, R₅, R₆, R₇, R₈ and R₁₁ are all hydrogen are prepared.

| Example | R₁₈ | R₁₉ | R₄ | R₃ | R₉ | R₁₀ |
|---|---|---|---|---|---|---|
| 33 | H | H | H | CF₃ | H | CF₃ |
| 34 | Methyl | Methyl | Cl | H | F | CF₃ |
| 35 | Methyl | Methyl | Cl | H | H | CF₃ |
| 36 | Cyclopropyl | | Cl | H | H | CF₃ |
| 37 | Cyclopropyl | | Cl | H | F | CF₃ |
| 38 | Methyl | Methyl | H | CF₃ | H | CF₃ |
| 39 | Methyl | Methyl | H | CF3 | F | CF₃ |

The NMR characteristics of these compounds are shown below :
- Example 33: CDCl₃ :: ¹H NMR 4.17 (2H, s) 6.75 (1H,s) 7.3-7.6 (8H, m) 8.71 (1H, s)
- Example 34: CDCl₃ :: ¹H NMR 1.79 (6H, s) 6.82 (1H, d) 7.2-7.6 (7H,m) 8.71 (1H, d)
- Example 35: CDCl₃ :: ¹H NMR 1.74 (6H, s) 6.78 (1H, d) 7.2-7.6(8H,m) 8.71 (1H, d)
- Example 36: CDCl₃ :: ¹H NMR 1.37 (2H, m) 1.81 (2H, m)6.35 (1H, d) 7.3-7.5 (8H, m) 8.61 (1H, d)
- Example 37: CDCl₃:: ¹H NMR 1.38 (2H, m) 1.82 (2H, m)6.35 (1H, d) 7.2-7.4 (7H, m) 8.59 (1H, d)
- Example 38: CDCl₃ :: ¹H NMR 1.78 (6H, s) 6.79 (1H, d) 7.3-7.6 (8H,m) 8.71 (1H, d)
- Example 39: CDCl_{3:}: ¹H NMR 1.74 (6H, s) 6.78 (1H, d) 7.2-7.4 (7H, m) 8.70 (1H, d)

### EXAMPLE 40

### Insecticidal And Acaricidal Evaluation of Test Compounds

The activity of these compounds is shown below:

| | TSM Twospotted mite | | | SAW southern army worm | | | |
|---|---|---|---|---|---|---|---|
| Example | 300 (ppm) | 100 (ppm) | 10 (ppm) | 3000 (ppm) | 1000 (ppm) | 300 (ppm) | 100 (ppm) |
| 23 | 9 | 9 | 0 | | | 1 | |
| 34 | 8 | 7 | 0 | | | 9 | 0 |
| 35 | 9 | 7 | 0 | | | 4 | |
| 36 | 9 | 6 | 0 | | | 0 | |
| 37 | 0 | | | 9 | | 0 | |
| 38 | 9 | 0 | 0 | | | 0 | |
| 39 | 0 | | | | | 8 | |

### EXAMPLE 41

### Preparation And Evaluation of 6-[(α,α,α,4-Tetrafluoro-m-tolyl)oxy]-4-pyrimidinyl α,α,α,-trifluoro-m-tolyl ketone

4-chloro-6-(3-trifluoromethylbenzyl) pyrimidine (0.30g, 1.1mmol), 4-fluoro-3-trifluoromethylphenol (0.24g, 1.3 mmol) and potassium carbonate (0.19g, 1.4 mmol) are combined in DMF (10ml) and heated at 70° C under a drying tube. The reaction is poured into ethyl acetate (50ml), washed with water, 2.5% sodium hydroxide, brine and dried over magnesium sulfate. The solvent is removed under vacuum and the residue chromatographed to obtain 0.1g of the title compound as a yellow oil. ¹H NMR CDCl₃ 7.63 (1H, d) 7.2 -8.4 (7H, m) 8.90 (1H, d).

The activity of this compound is measured as described above and the results obtained are shown below:

| | TSM two spotted mite | | | SAW southern army worm | | | |
|---|---|---|---|---|---|---|---|
| Example | 300 (ppm) | 100 (ppm) | 10 (ppm) | 1000 (ppm) | 300 (ppm) | 100 (ppm) | 10 (ppm) |
| 41 | 9 | 8 | 5 | | 0 | | |

## Claims

1. A compound of Formula I 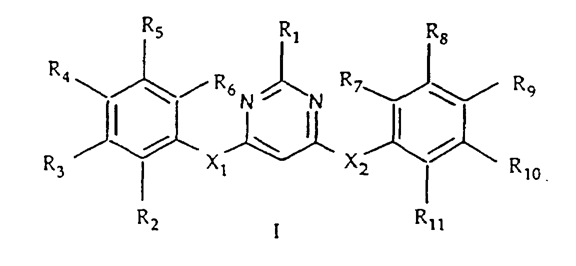 wherein
R₁ is hydrogen, (C₁-₆)alkylthio, halo(C₁-₆)alkylthio (C₁-₆)alkylsulphinyl, halo(C₁-₆)alkylsulphinyl, (C₁₋₆)alkylsulphonyl, halo(C₁-₆)alkylsulphonyl or NR₁₂R₁₃;
R₁₂ and R₁₃ are each independently hydrogen, (C₁-₆)alkyl, or halo(C₁-₆)alkyl;
R₂, R₃, R₄, R₅, R₆, R₇, R₈, R₉ and R₁₁ are each independently selected from hydrogen, halogen, cyano, nitro, (C₁-₆)alkyl, halo(C₁-₆)alkyl, (C₁-₆)alkoxy, halo (C₁-₆)alkoxy, (C₁-₆)alkoxy(C₁-₆)alkyl, halo(C₁-₆)alkoxy(C₁-₆)alkyl, (C₁-₆)alkylthio, halo(C₁-₆)alkylthio, (C₁-₆)alkyl sulphinyl, (C₁-₆)alkylsulphinyl, (C₁-₆)alkylsulphonyl, halo(C₁-₆)alkylsulphonyl, amino, (C₁-₆)alkylamino, di(C₁-₆)alkylamino, halo(C₁-₆)alkylamino, (C₁-₆)alkylhaloalkyl (C₁-₆)amino, dihalo(C₁-₆)alkylamino,(C₁-₆)alkyloxy-carbonyl, halo(C₁-₆)alkyloxycarbonyl, (C₂-₆)alkenyl, halo(C₂-₆)alkenyl, (C₂-₆)alkynyl, halo(C₂-₆)alkynyl;
R₁₀ is halogen, halo(C₁-₆)alkyl, halo(C₂-₆)alkenyl or halo(C₂-₆)alkynyl;
and X₁ and X₂ are independently selected from the group consisting of NR₁₄, NR₁₅, O, CH₂, CR₁₈R₁₉, CO, C=NOR₂₀;
wherein R₁₄ is (C₁-₆)alkyl;
R₁₅ is H, (C₁-₆)alkyl or CH(OR₁₆) (OR₁₇) ;
R16 and R₁₇ are each independently (C₁-₆)alkyl;
R₁₈ and R₁₉ each independently selected from H, (C₁-₆)alkyl, (C₂-₆)alkenyl and (C₂-₆)alkynyl which are all optionally substituted by one or more halogens which are the same or different, or R₁₈ and R₁₉ may together form a carbocyclic ring which is optionally substituted by one or more halogens which are the same or different;
R₂₀ is (C₁-₆)alkyl ;
with the proviso that X₁ cannot be the same as X₂.

2. The compound according to claim 1 wherein
a) one of X₁ and X₂ is NR₁₄ and the other is O or S;
b) one of X₁ and X₂ is NR₁₅ and the other is CH₂;
c) one of X, and X₂ is CR₁₈R₁₉ or CO and the other is O; or
d) one of X₁ and X₂ is C=NOR₂₀ and the other is CH₂.

3. The compound according to Claim 1 wherein X₁ is NR₁₄ and X₂ is O.

4. The compound according to Claim 1 wherein R₁₀ is chlorine, halo(C₁-₄)alkyl or halovinyl.

5. The compound according to Claim 1 wherein R₃ is halogen, halo(C₁-₆)alkyl, halo(C₂-₆)alkenyl or halo(C₂-₆)alkynyl.

6. The compound according to Claim 1 selected from the group consisting of:
4-(α,α,α,4-Tetrafluoro-n-methyl-m-toluidino)-6-[(α,α,α,4-tetrafluoro-m-tolyl)oxy]pyrimidine ;
4-(α,α,α,,4-Trifluoro-N-methyl-m-toluidino)-6-[(α,α,α,-trifluoro-m-tolyl)oxy]pyrimidine;
4-[(α,α,α,4-Tetrafluoro-m-tolyl)oxy]-6-(α,α,α,-trifluoro-N-methyl-m-toluidino)pyrimidine;
4-(3,4-Dichlorophenyoxy)-6-(α,α,α,-trifluoro-N-methyl-m-toluidino)pyrimidine;
4-[(4-Chloro-α,α,α,-trifluoro-m-tolyl)oxy]-6-(a,a,a,trifluoro-N-methyl-m-toluidino)pyrimidine;
4- (α,α,α,4-Tetrafluoro-N-methyl-m-toluidino) -6-[(α,α,α,trifluoro-m-tolyl)oxy]pyrimidine;
4- [(4-Chloro-α,α,α,-trifluoro-m-toly)oxy]-6-(α,α,α,,4-tetrafluoro-N-methyl-m-toluidino)pyrimidine;
4-(4-Chloro-α,α,α,-trifluoro-N-methyl-m-toluidino)-6-[(α,α,α,4-tetrafluoro-m-tolyl)oxy]pyrimidine;
4- (3,4-Dichloro-N-methylanilino)-6-[(α,α,α,-trifluoro-m-tolyl)oxy]pyrimidine;
4-(3,4-Dichloro-N-methylanilino)-6-(α,α,α,-4-tetrafluoro-m-tolyl)oxy]pyrimidine;
4-(3,4-Dichloro-N-methylanilino)-6-(3,4-dichlorophenoxy)-pyrimidine;
4-(α,α,α,4-Tetrafluoro-m-toluidino)-6-[m-(trifluoromethyl)benzyl]-, hydrochloride;
4-(3,4-Dichlorobenzyl)-6-(α,α,α,4-tetrafluoro-m-toluidino) hydrochloride;
4-(4-Chloro-α,α,α,-trifluoro-m-toluidino)-6-[m-(trifluoromethyl)benzyl]-, hydrochloride;
4-[m-(Trifluoromethyl)benzyl]-6-(α,α,α,-trifluoro-m-toluidino)pyrimidine, hydrochloride;
4-(α,α,α,4-Tetrafluoro-N-methyl-m-toluidino)-6-[m-trifluoromethyl)benzyl]pyrimidine;
4-(4-Chloro-α,α,α,-trifluoro-N-methyl-m-toluidino)-6-(3,4-dichlorobenzyl)pyrimidine;
4-(3,4-Dichlorobenzyl)-6-(α,α,α,,4-tetrafluoro-N-methyl-m-toluidino)pyrimidine;
4-(4-Chloro-α,α,α,-trifluoro-m-toluidino)-6-(3,4-dichlorobenzyl)pyrimidine ;
4-(3,4-Dichlorobenzyl)-6-[N-diethoxymethyl)-α,α,α,,4-tetrafluoro-m-toluidino]pyrimidine;
4-(4-Chloro-α,α,α,-trifluoro-N-methyl-m-toluidino)-6-[m-(trifluoromethyl)benzyl]pyrimidine;
4-(3,4-Dichlorobenzyl) -6-(α,α,α,-trifluoro-m-toluidino)pyrimidine;
4-(3,4-Dichlorobenzyl)-6-(α,α,α,-trifluoro-N-methyl-m-toluidino)pyrimidine;
6-(α,α,α,4-Tetrafluoro-m-toluidino)-4- [4-fluoro-3-(trifluoromethyl)benzyl]pyrimidine;
4-[4-Fluoro-3-(trifluoromethyl)benzyl]-6-(α,α,α,4-tetrafluoro-N-methyl-m-toluidino)pyrimidine;
4-(4-Chloro-α,α,α,-trifluoro-m-toluidino)-6-[4-fluoro-3-(trifluoromethyl)benzyl]pyrimidine ;
4-(4-Chloro-α,α,α,-trifluoro-N-methyl-m-toluidino)-6-[4-fluoro-3-(trifluoromethyl)benzyl]pyrimidine;
4-[m-(Trifluoromethyl)benzyl]-6-[(α,α,α,-trifluoro-m-tolyl)oxy]pyrimidine;
4-(p-Chloro-α,α-dimethylbenzyl) -6-[(α,α,α,4-tetrafluoro-m-tolyl)oxy]pyrimidine;
4-(p-Chloro-α,α-dimethylbenzyl)-6-[(α,α,α,4-trifluoro-m-tolyl)oxy]pyrimidine;
4-[1-(p-Chlorophenyl)cyclopropyl)-6-[(α,α,α,-trifluoro-m-tolyl)oxy]pyrimidine;
4-[1-(p-Chlorophenyl)cyclopropyl] -6-[(α,α,α,4-tetrafluoro-m-tolyl)oxy]pyrimidine;
Ketone, 6-[(α,α,α,4-tetrafluoro-m-tolyl)oxy]-4-pyrimidinyl α,α,α,4-trifluoro-m-tolyl ;
4-[α,α-Dimethyl-m-(trifluoromethyl)benzyl]-6-[(α,α,α,-trifluoro-m-tolyl)oxy]pyrimidine;
4-[α,α-Dimethyl-m-(trifluoromethyl)benzyl]-6-[(α,α,α,4-tetrafluoro-m-tolyl)oxy]pyrimidine; and
6-[m-(Trifluoromethyl)benzyl]-4-pyrimidinyl α,α,α,-trifluoro-m-tolyl ketone, O-methyloxime, (E)- and (Z) -.

7. The compound according to Claim 1 wherein R₁ is (C₁₋₆)alkylthio, halo(C₁₋₆)alkylthio, (C₁₋₆)alkylsulfinyl, halo(C₁₋₆)alkylsulfinyl, (C₁₋₆)alkylsulfoxyl, halo(C₁₋ ₆)alkyl-sulfoxyl or NR₁₂R₁₃; and one of X₁ and X₂ is NR₁₄ and the other is O.

8. A method for the control of acarina or insects which comprises contacting said acarid or insect or their habitats, breeding grounds or locus with a acaricidally or insecticidally effective amount of a compound described in any one of claims 1-7.

9. A composition which comprises an agriculturally acceptable carrier and a pesticidally effective amount of a compound described in any one of claims 1-7.

10. A process for the preparation of a compound of formula I wherein X₁, X₂, R₁, R₂, R₃, R₄, R₅, R₆, R₇, R₈, R₉, R₁₀ and R₁₁ are as described in any one of claims 1-7 which comprises the following steps:
i) reacting a compound of formula IV 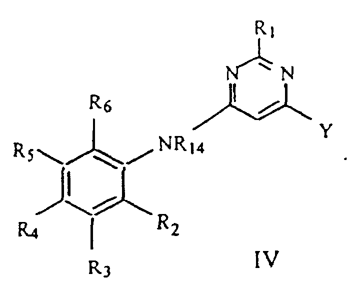 with a phenol of formula V or thiophenol of formula V¹ 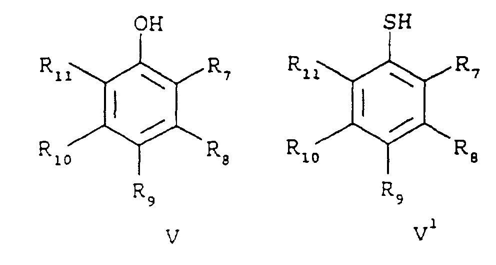 to provide a compound of formula I wherein X₁ is NR₁₄ and X₂ is O or S;
ii) reacting a compound of formula IV^{a} 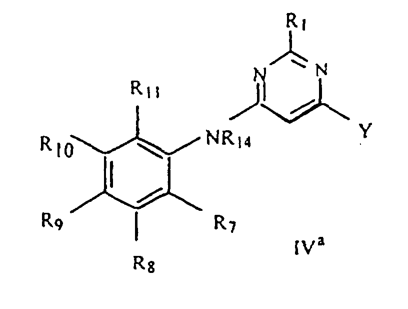 with a phenol of formula V^{a} or thiophenol of formula V^{1a} 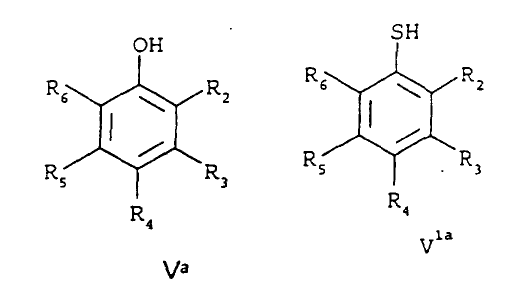 to provide a compound of formula I wherein X₁ is O or S and X₂ is NR₁₄;
iii) reacting a compound of formula X 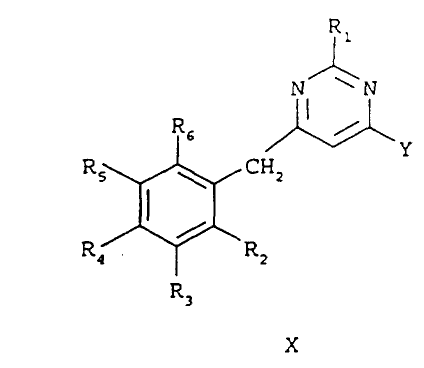 wherein Y is a halogen, with the appropriate aniline derivative of formula XI 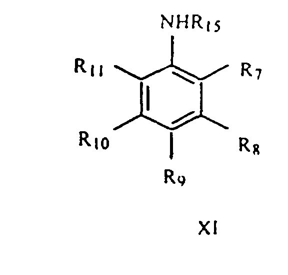 to provide a compound of formula I wherein X₁ is CH₂ and X₂ is NR₁₅;
iv) reacting a compound of formula X^{a} 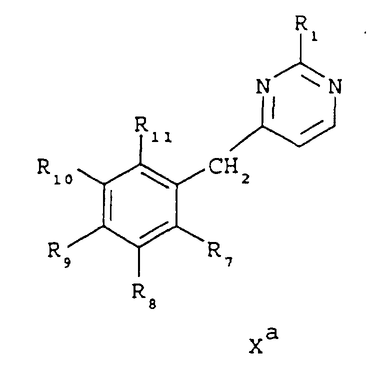 wherein Y is a halogen, with the appropriate aniline derivative of formula XI^{a} 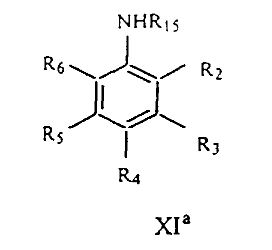 to provide a compound of formula I wherein X₁ is NR₁₅ and X₂ is CH₂;
v) reducing a compound of formula XVIII 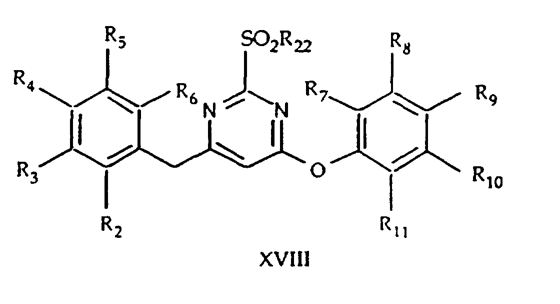 to provide a compound of formula I wherein X₁ is CR₁₈R₁₉, R₁₈ and R₁₉ are both hydrogen, X₂ is O and R₁ is hydrogen;
vi) reducing a compound of formula XVIII^{a} 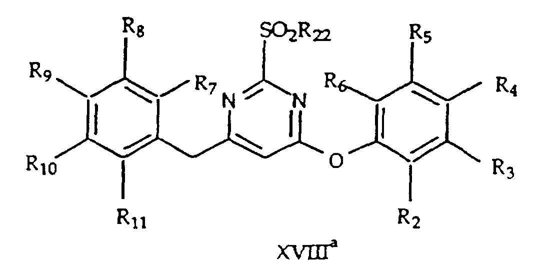 to provide a compound of formula I wherein X₁ is O, X₂ is CR₁₈R₁₉, R₁₈ and R₁₉ are both hydrogen and R₁ is hydrogen;
vii) reacting a compound of formula XVIII with an amine of formula XIX:
NHR₁₂R₁₃ XIX
to provide a compound of formula I wherein X₁ is CR₁₈R₁₉, R₁₈ and R₁₉ are both hydrogen, X₂ is O and R₁ is NR₁₂R₁₃;
viii) reacting a compound of formula XVIII^{a} with an amine of formula XIX, as described above, to provide a compound of formula I wherein X₁ is O, X₂ is CR₁₈R₁₉, R₁₈ and R₁₉ are both hydrogen and R₁ is NR₁₂R₁₃;
ix) reacting a compound of formula XXIV 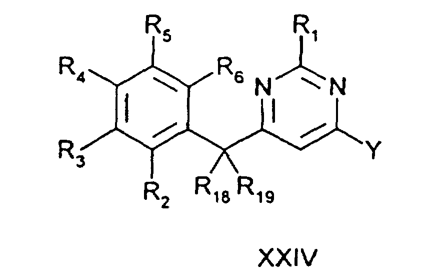 wherein Y is a halogen, with a phenol of formula V 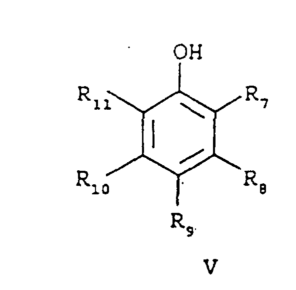 to provide a compound of formula I wherein X₁ is CR₁₈R₁₉, X₂ is O and R₁₈ and R₁₉ are each selected from (C₁-₆)alkyl, (C₂₋₆)alkenyl and (C₂₋₆)alkynyl which are all optionally substituted by one or more halogens which are the same or different, or R₁₈ and R₁₉ may together form a carbocyclic ring which is optionally substituted by one or more halogens which are the same or different;
x) reacting a compound of formula XXIV^{a} 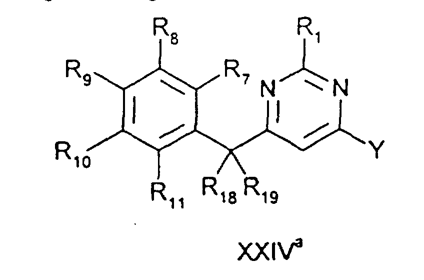 wherein Y is a halogen, with a phenol of formula V^{a} 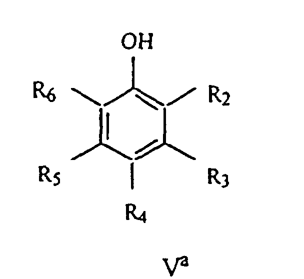 to provide a compound wherein X₁ is O, X₂ is CR₁₈R₁₉, and R₁₈ and R₁₉ are each selected from (C₁-₆)alkyl, (C₂-₆)alkenyl and (C₂-₆)alkynyl which are all optionally substituted by one or more halogens which are the same or different, or R₁₈ and R₁₉ may together form a carbocyclic ring which is optionally substituted by one or more halogens which are the same or different;
xi) oxidizing a compound of formula I wherein X₁ is CH₂ and X₂ is O to the corresponding compound wherein X₁ is CO;
xii) oxidizing a compound of formula I wherein X₂ is CH₂ and X₁ is O to the corresponding compound wherein X₂ is CO;
xiii) oximizing a compound of formula XXIX 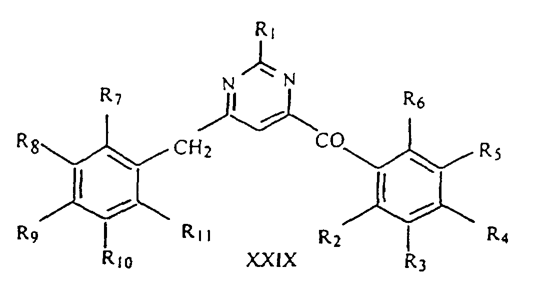 to the corresponding oxime to provide a compound of formula I wherein X₁ is C=NOR₂₀ and X₂ is CH₂; or
xiv) oximizing a compound of formula XXIX^{a} 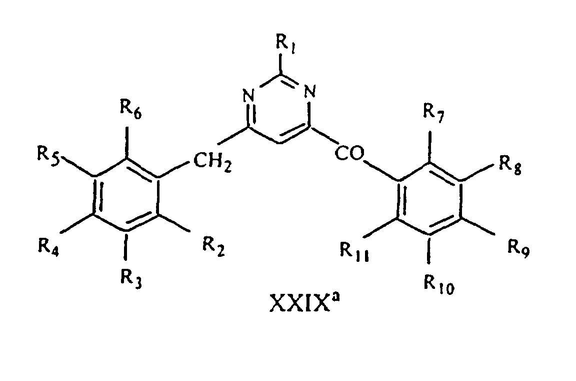 to the corresponding oxime to provide a compound of formula I wherein X₁ is CH₂ and X₂ is C=NOR_{20.}

## Patentansprüche

1. Verbindungen der Formel I 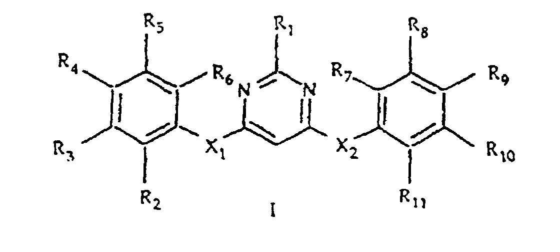 in welcher
R₁ für Wasserstoff, (C₁₋₆)-Alkylthio, Halogen-(C₁₋₆)-alkylthio, (C₁₋₆) -Alkylsulfinyl, Halogen- (C₁₋₆) -alkylsulfinyl, (C₁₋₆)-Alkylsulfonyl, Halogen-(C₁₋₆)-alkylsulfonyl oder NR₁₂R₁₃ steht;
R₁₂ und R₁₃ jeweils unabhängig für Wasserstoff, (C₁₋₆)-Alkyl oder Halogen-(C₁₋₆)-alkyl stehen;
R₂, R₃, R₄, R₅, R₆, R₇, R₈, R₉ und R₁₁ jeweils unabhängig aus Wasserstoff, Halogen, Cyano, Nitro, (C₁₋₆)-Alkyl, Halogen-(C₁₋₆)-alkyl, (C₁₋₆)-Alkoxy, Halogen-(C₁₋₆)-alkoxy, (C₁₋₆)-Alkoxy-(C₁₋₆)-alkyl, Halogen-(C₁₋₆)-alkoxy-(C₁₋₆)-alkyl, (C₁₋₆)-Alkylthio, Halogen-(C₁₋₆)-alkylthio, (C₁₋₆)-Alkylsulfinyl, Halogen-(C₁₋₆)-alkylsulfinyl, (C₁₋₆)-Alkylsulfonyl, Halogen-(C₁₋₆)-alkylsulfonyl, Amino, (C₁₋₆)-Alkylamino, Di-(C₁₋₆)-alkylamino, Halogen-(C₁₋₆)-alkylamino, (C₁₋₆)-Alkyl-halogen-(C₁₋₆)-alkylamino, Dihalogen-(C₁₋₆)-alkylamino, (C₁₋₆)-Alkyloxycarbonyl, Halogen-(C₁₋₆)-alkyloxycarbonyl, (C₂₋₆)-Alkenyl, Halogen-(C₂₋₆)-alkenyl, (C₂₋₆)-Alkinyl, Halogen-(C₂₋₆)-alkinyl ausgewählt sind;
R₁₀ für Halogen, Halogen-(C₁₋₆)-alkyl, Halogen(C₂₋₆)-alkenyl oder Halogen-(C₂₋₆)-alkinyl steht;
und X₁ und X₂ unabhängig aus der Gruppe NR₁₄, NR₁₅, O, CH₂, CR₁₈R₁₉, CO, C=NOR₂₀ ausgewählt sind;
wobei R₁₄ für (C₁₋₆)-Alkyl steht;
R₁₅ für H, (C₁₋₆)-Alkyl oder CH(OR₁₆) (OR₁₇) steht;
R₁₆ und R₁₇ jeweils unabhängig für (C₁₋₆)-Alkyl stehen;
R₁₈ und R₁₉ jeweils unabhängig aus H, (C₁₋₆)-Alkyl, (C₂₋₆)-Alkenyl und (C₂₋₆)-Alkinyl ausgewählt sind, die jeweils gegebenenfalls durch ein oder mehrere gleiche oder verschiedene Halogene substituiert sind, oder R₁₈ und R₁₉ zusammen einen carbocyclischen Ring bilden können, der gegebenenfalls durch ein oder mehrere gleiche oder verschiedene Halogene substituiert ist;
R₂₀ für (C₁₋₆)-Alkyl steht;
mit der Maßgabe, daß X₁ nicht dieselbe Bedeutung wie X₂ haben kann.

2. Verbindungen nach Anspruch 1, wobei
a) einer der Reste X₁ und X₂ für NR₁₄ und der andere für O oder S steht;
b) einer der Reste X₁ und X₂ für NR₁₅ und der andere für CH₂ steht;
c) einer der Reste X₁ und X₂ für CR₁₈R₁₉ oder CO und der andere für O steht; oder
d) einer der Reste X₁ und X₂ für C=NOR₂₀ und der andere für CH₂ steht.

3. Verbindungen nach Anspruch 1, wobei X₁ für NR₁₄ und X₂ für O steht.

4. Verbindungen nach Anspruch 1, wobei R₁₀ für Chlor, Halogen-(C₁₋₄)-alkyl oder Halogenvinyl steht.

5. Verbindungen nach Anspruch 1, wobei R₃ für Halogen, Halogen-(C₁₋₆)-alkyl, Halogen-(C₂₋₆)-alkenyl oder Halogen-(C₂₋₆)-alkinyl steht.

6. Verbindungen nach Anspruch 1, ausgewählt aus der Gruppe:
4-(α,α,α,4-Tetrafluor-n-methyl-m-toluidino)-6-[(α,α,α,4-tetrafluor-m-tolyl)oxy]pyrimidin;
4-(α,α,α,4-Trifluor-N-methyl-m-toluidino)-6-[(α,α,α-trifluor-m-tolyl)oxy]pyrimidin;
4-[(α,α,α,4-Tetrafluor-m-tolyl)oxy]-6-(α,α,α-trifluor-N-methyl-m-toluidino)pyrimidin;
4-(3,4-Dichlorphenyloxy)-6-(α,α,α-trifluor-N-methyl-m-toluidino)pyrimidin;
4-[(4-Chlor-α,α,α-trifluor-m-tolyl)oxy]-6-(α,α,α-trifluor-N-methyl-m-toluidino)pyrimidin;
4-(α,α,α,4-Tetrafluor-N-methyl-m-toluidino)-6-[(α,α,α-trifluor-m-tolyl)oxy]pyrimidin;
4-[(4-Chlor-α,α,α-trifluor-m-tolyl)oxy]-6-(α,α,α,4-tetrafluor-N-methyl-m-toluidino)pyrimidin;
4-(4-Chlor-α,α,α-trifluor-N-methyl-m-toluidino)-6-[(α,α,α,4-tetrafluor-m-tolyl)oxy]pyrimidin;
4-(3,4-Dichlor-N-methylanilino)-6-[(α,α,α-trifluor-m-tolyl)oxy]pyrimidin;
4-(3,4-Dichlor-N-methylanilino)-6-[(α,α,α-4-tetrafluor-m-tolyl)oxy]pyrimidin;
4-(3,4-Dichlor-N-methylanilino)-6-(3,4-dichlorphenoxy)-pyrimidin;
4-(α,α,α,4-Tetrafluor-m-toluidino)-6-[m-(trifluormethyl)benzyl]-hydrochlorid;
4-(3,4-Dichlorbenzyl)-6-(α,α,α, 4-tetrafluor-m-toluidino)-hydrochlorid;
4-(4-Chlor-α,α,α-trifluor-m-toluidino)-6-[m-(trifluormethyl)benzyl] -hydrochlorid;
4-[m-(Trifluormethyl)benzyl]-6-(α,α,α-trifluor-m-toluidino)pyrimidin-hydrochlorid;
4-(α,α,α,4-Tetrafluor-N-methyl-m-toluidino)-6-[m-trifluormethyl)benzyl]pyrimidin;
4-(4-Chlor-α,α,α-trifluor-N-methyl-m-toluidino)-6-(3,4-dichlorbenzyl)pyrimidin;
4- (3,4-Dichlorbenzyl)-6-(α,α,α,4-tetrafluor-N-methyl-m-toluidino)pyrimidin;
4-(4-Chlor-α,α,α-trifluor-m-toluidino)-6-(3,4-dichlorbenzyl)pyrimidin;
4-(3,4-Dichlorbenzyl)-6-[N-diethoxymethyl)-α,α,α,4-tetrafluor-m-toluidino]pyrimidin;
4-(4-Chlor-α,α,α-trifluor-N-methyl-m-toluidino)-6-[m-(trifluormethyl)benzyl]pyrimidin;
4-(3,4-Dichlorbenzyl)-6-(α,α,α-trifluor-m-toluidino)-pyrimidin;
4-(3,4-Dichlorbenzyl)-6-(α,α,α-trifluor-N-methyl-m-toluidino)pyrimidin;
6-(α,α,α,4-Tetrafluor-m-toluidino)-4-[4-fluor-3-(trifluormethyl)benzyl]pyrimidin ;
4-[4-Fluor-3-(trifluormethyl)benzyl]-6-(α,α,α,4-tetrafluor-N-methyl-m-toluidino)pyrimidin;
4-(4-Chlor-α,α,α-trifluor-m-toluidino)-6-[4-fluor-3-(trifluormethyl)benzyl]pyrimidin;
4-(4-Chlor-α,α,α,-trifluor-N-methyl-m-toluidino)-6-[4-fluor-3-(trifluormethyl)benzyl]pyrimidin;
4-[m-(Trifluormethyl)benzyl]-6-[(α,α,α,-trifluor-m-tolyl)oxy]pyrimidin;
4-(p-Chlor-α,α-dimethylbenzyl)-6-[(α,α,α,4-tetrafluor-m-tolyl)oxy]pyrimidin;
4-(p-Chlor-α,α-dimethylbenzyl)-6-[(α,α,α, 4-trifluor-m-tolyl)oxy]pyrimidin;
4-[1-(p-Chlorphenyl)cyclopropyl]-6-[(α,α,α-trifluor-m-tolyl)oxy]pyrimidin;
4-[1-(p-Chlorphenyl)cyclopropyl]-6-[(α,α,α,4-tetrafluor-m-tolyl)oxy]pyrimidin;
6-[(α,α,α,4-Tetrafluor-m-tolyl)oxy]-4-pyrimidinyl-α,α,α,4-trifluor-m-tolyl-keton;
4-[α,α-Dimethyl-m-(trifluormethyl)benzyl]-6-[(α,α,α-trifluor-m-tolyl)oxy]pyrimidin;
4-[α,α-Dimethyl-m-(trifluormethyl)benzyl]-6-[(α,α,α,4-tetrafluor-m-tolyl)oxy]pyrimidin; und
(E)- und (Z)-6-[m-(Trifluormethyl)benzyl]-4-pyrimidinyl-α,α,α-trifluor-m-tolyl-keton-0-methyloxim.

7. Verbindungen nach Anspruch 1, wobei R₁ für (C₁₋₆)-Alkylthio, Halogen-(C₁₋₆)-alkylthio, (C₁₋₆) -Alkylsulfinyl, Halogen- (C₁₋₆) -alkylsulfinyl, (C₁₋₆)-Alkylsulfoxyl, Halogen-(C₁₋₆)-alkylsulfoxyl oder NR₁₂R₁₃ steht; und einer der Reste X₁ und X₂ für NR₁₄ und der andere für O steht.

8. Verfahren zur Bekämpfung von Akarinen oder Insekten, bei dem man die Akarine oder das Insekt oder ihre Lebensräume, Brutplätze oder Ort, an dem sie vorkommen, mit einer akarizid oder Insektizid wirksamen Menge einer der in einem der Ansprüche 1-7 beschriebenen Verbindungen in Kontakt bringt.

9. Zusammensetzung, enthaltend einen landwirtschaftlich unbedenklichen Trägerstoff und eine pestizid wirksame Menge einer der in einem der Ansprüche 1-7 beschriebenen Verbindungen.

10. Verfahren zur Herstellung einer Verbindung der Formel I, wobei X₁, X₂, R₁, R₂, R₃, R₄, R₅, R₆, R₇, R₈, R₉, R₁₀ und R₁₁ wie in einem der Ansprüche 1-7 beschrieben sind, welches die folgenden Schritte umfaßt:
i) Umsetzung einer Verbindung der Formel IV 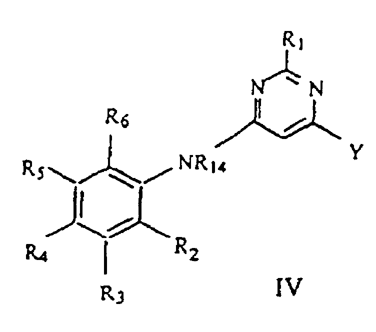 mit einem Phenol der Formel V oder einem Thiophenyl der Formel V¹ 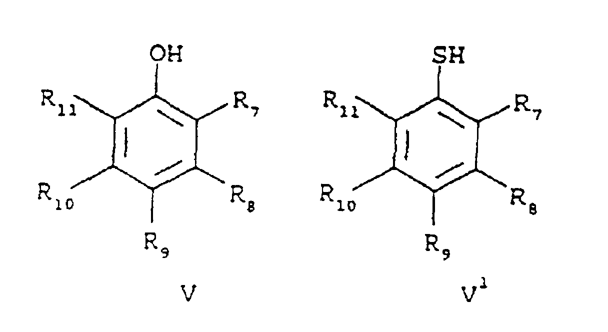 zu einer Verbindung der Formel I, wobei X₁ für NR₁₄ und X₂ für O oder S steht;
(ii) Umsetzung einer Verbindung der Formel IV^{a} 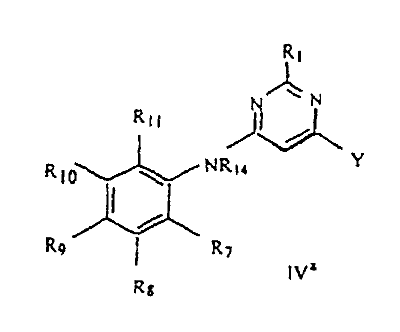 mit einem Phenol der Formel V^{a} oder einem Thiophenyl der Formel V^{1a} 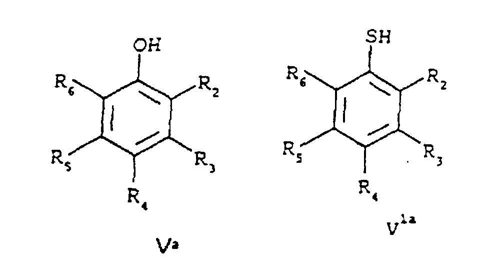 zu einer Verbindung der Formel I, wobei X₁ für O oder S und X₂ für NR₁₄ steht;
iii) Umsetzung einer Verbindung der Formel X 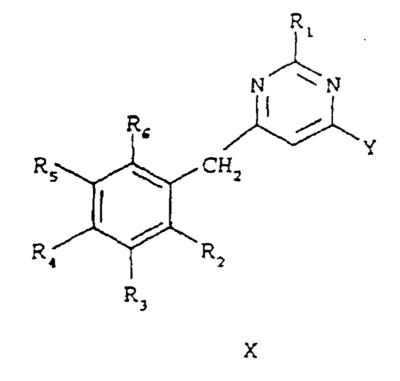 wobei Y für Halogen steht, mit dem entsprechenden Anilinderivat der Formel XI 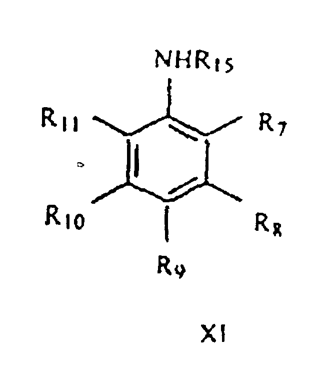 zu einer Verbindung der Formel I, wobei X₁ für CH₂ und X₂ für NR₁₅ steht;
iv) Umsetzung einer Verbindung der Formel X^{a} 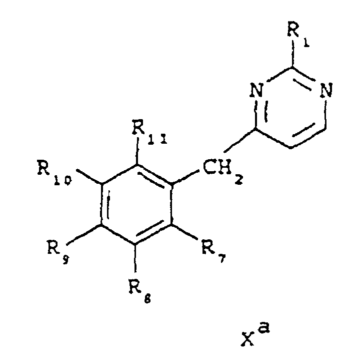 wobei Y für Halogen steht, mit dem entsprechenden Anilinderivat der Formel XI^{a} 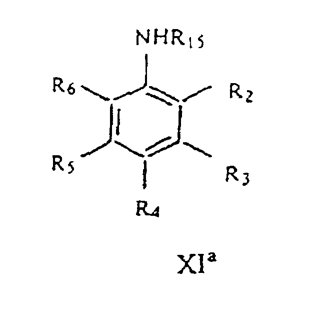 zu einer Verbindung der Formel I, wobei X₁ für NR₁₅ und X₂ für CH₂ steht;
v) Reduktion einer Verbindung der Formel XVIII 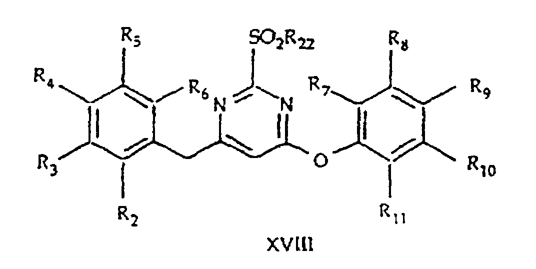 zu einer Verbindung der Formel I, wobei X₁ für CR₁₈R₁₉ steht, R₁₈ und R₁₉ jeweils für Wasserstoff stehen, X₂ für O und R₁ für Wasserstoff steht;
vi) Reduktion einer Verbindung der Formel XVIII^{a} 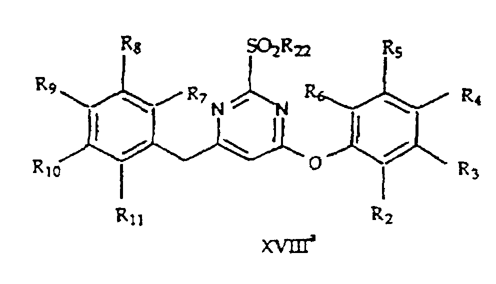 zu einer Verbindung der Formel I, wobei X₁ für O und X₂ für CR₁₈R₁₉ steht, R₁₈ und R₁₉ jeweils für Wasserstoff stehen und R₁ für Wasserstoff steht;
vii) Umsetzung einer Verbindung der Formel XVIII mit einem Amin der Formel XIX:
NHR₁₂R₁₃ XIX
zu einer Verbindung der Formel I, wobei X₁ für CR₁₈R₁₉ steht, R₁₈ und R₁₉ jeweils für Wasserstoff stehen, X₂ für O und R₁ für NR₁₂R₁₃ steht;
viii) Umsetzung einer Verbindung der Formel XVIII^{a} mit einem Amin der Formel XIX, wie oben beschrieben, zu einer Verbindung der Formel I, wobei X₁ für O und X₂ für CR₁₈R₁₉ steht, R₁₈ und R₁₉ jeweils für Wasserstoff stehen und R₁ für NR₁₂R₁₃ steht;
ix) Umsetzung einer Verbindung der Formel XXIV 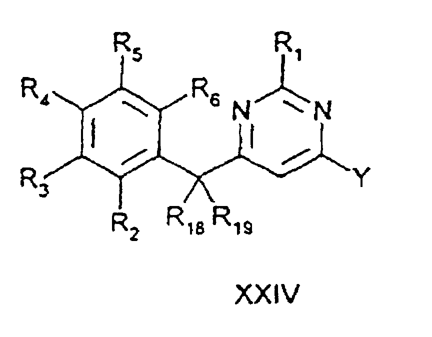 wobei Y für Halogen steht, mit einem Phenol der Formel V 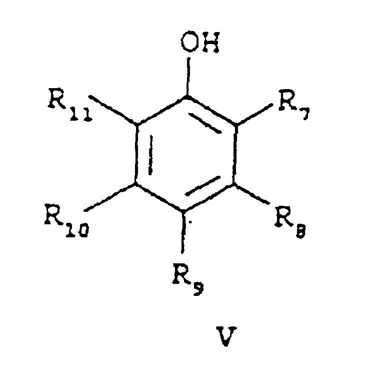 zu einer Verbindung der Formel I, wobei X₁ für CR₁₈R₁₉ und X₂ für O steht und R₁₈ und R₁₉ jeweils aus (C₁₋₆)-Alkyl, (C₂₋₆)-Alkenyl und (C₂₋₆)-Alkinyl ausgewählt sind, die jeweils gegebenenfalls durch ein oder mehrere gleiche oder verschiedene Halogene substituiert sind, oder R₁₈ und R₁₉ zusammen einen carbocyclischen Ring bilden können, der gegebenenfalls durch ein oder mehrere gleiche oder verschiedene Halogene substituiert ist;
x) Umsetzung einer Verbindung der Formel XXIV^{a} 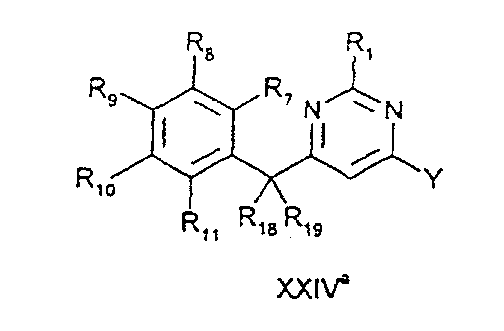 wobei Y für Halogen steht, mit einem Phenol der Formel V^{a} 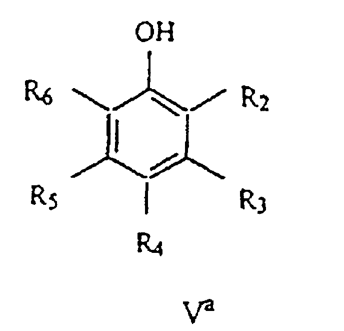 zu einer Verbindung, in welcher X₁ für O und X₂ für CR₁₈R₁₉ steht und R₁₈ und R₁₉ jeweils aus (C₁₋₆)-Alkyl, (C₂₋₆)-Alkenyl und (C₂₋₆) -Alkinyl ausgewählt sind, die jeweils gegebenenfalls durch ein oder mehrere gleiche oder verschiedene Halogene substituiert sind, oder R₁₈ und R₁₉ zusammen einen carbocyclischen Ring bilden können, der gegebenenfalls durch ein oder mehrere gleiche oder verschiedene Halogene substituiert ist;
xi) Oxidieren einer Verbindung der Formel I, wobei X₁ für CH₂ und X₂ für O steht, zur entsprechenden Verbindung, in welcher X₁ für CO steht;
xii) Oxidieren einer Verbindung der Formel I, wobei X₂ für CH₂ und X₁ für O steht, zur entsprechenden Verbindung, in welcher X₂ für CO steht;
xiii) Oximieren einer Verbindung der Formel XXIX 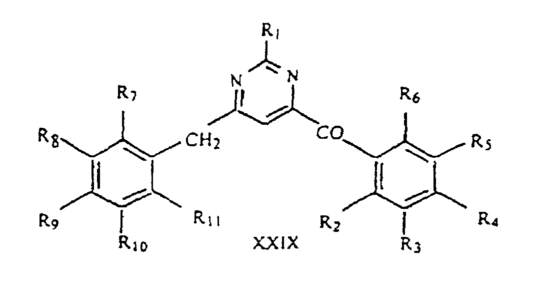 zum entsprechenden Oxim, wodurch man eine Verbindung der Formel I erhält, in welcher X₁ für C=NOR₂₀ und X₂ für CH₂ steht; oder
xiv) Oximieren einer Verbindung der Formel XXIX^{a} 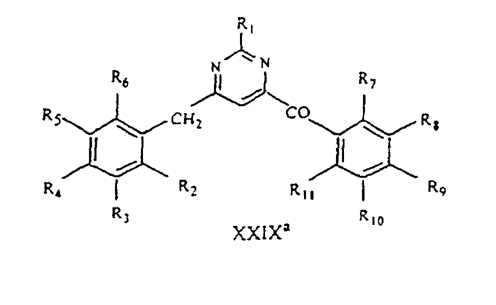 zum entsprechenden Oxim, wodurch man eine Verbindung der Formel I erhält, in welcher X₁ für CH₂ und X₂ für C=NOR₂₀ steht.

## Revendications

1. Composé de Formule I 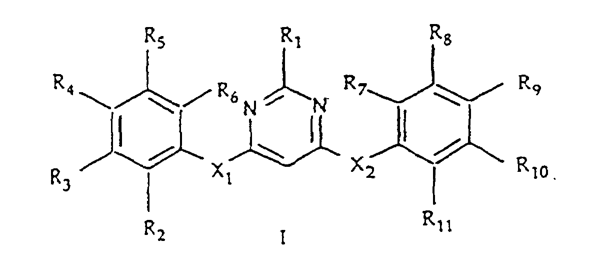 dans laquelle
R₁ est l'hydrogène ou un groupe alkylthio en C₁-C₆, halogénoalkylthio en C₁-C₆, alkylsulfinyle en C₁-C₆, halogénoalkylsulfinyle en C₁-C₆, alkylsulfonyle en C₁-C₆, halogénoalkylsulfonyle en C₁-C₆ ou NR₁₂R₁₃ ;
R₁₂ et R₁₃ sont chacun indépendamment l'hydrogène, un groupe alkyle en C₁-C₆ ou un groupe halogénoalkyle en C₁-C₆ ;
R₂, R₃, R₄, R₅, R₆, R₇, R₈, R₉ et R₁₁ sont choisis chacun indépendamment parmi l'hydrogène, les halogènes et les groupes cyano, nitro, alkyle en C₁-C₆, halogénoalkyle en C₁-C₆, alcoxy en C₁-C₆, halogénoalcoxy en C₁-C₆, (alcoxy en C₁-C₆)alkyle en C₁-C₆, (halogénoalcoxy en C₁-C₆)alkyle en C₁-C₆, alkylthio en C₁-C₆, halogénoalkylthio en C₁-C₆, alkylsulfinyle en C₁-C₆, halogénoalkylsulfinyle en C₁-C₆, alkylsulfonyle en C₁-C₆, halogénoalkylsulfonyle en C₁-C₆, amino, alkylamino en C₁-C₆, di (alkyle en C₁-C₆)amino, halogénoalkylamino en C₁-C₆, (alkyle en C₁-C₆)(halogénoalkyle en C₁-C₆)amino, di(halogénoalkyle en C₁-C₆)amino, (alkyle en C₁-C₆)oxycarbonyle, (halogénoalkyle en C₁-C₆)oxycarbonyle, alcényle en C₂-C₆, halogénoalcényle en C₂-C₆, alcynyle en C₂-C₆, halogénoalcynyle en C₂-C₆ ;
R₁₀ est un halogène ou un groupe halogénoalkyle en C₁-C₆, halogénoalcényle en C₂-C₆ ou halogénoalcynyle en C₂-C₆ ; et
X₁ et X₂ sont choisis indépendamment dans la classe formée par NR₁₄, NR₁₅, O, CH₂, CR₁₈R₁₉, CO, C=NOR₂₀ ; où
R₁₄ est un groupe alkyle en C₁-C₆ ;
R₁₅ est H, un groupe alkyle en C₁-C₆ ou CH(OR₁₆) (OR₁₇) ;
R₁₆ et R₁₇ sont chacun indépendamment un groupe alkyle en C₁-C₆ ;
R₁₈ et R₁₉ sont choisis chacun indépendamment parmi H et les groupes alkyle en C₁-C₆, alcényle en C₂-C₆ et alcynyle en C₂-C₆ qui sont tous facultativement substitués par un ou plusieurs halogènes identiques ou différents, ou bien R₁₈ et R₁₉ peuvent former ensemble un carbocycle qui est facultativement substitué par un ou plusieurs halogènes identiques ou différents ;
R₂₀ est un groupe alkyle en C₁-C₆ ;
à condition que X₁ ne soit pas identique à X₂.

2. Composé selon la revendication 1, dans lequel
a) l'un de X₁ et X₂ est NR₁₄ et l'autre est O ou S ;
b) l'un de X₁ et X₂ est NR₁₅ et l'autre est CH₂ ;
c) l'un de X₁ et X₂ est CR₁₈R₁₉ ou CO et l'autre est O ; ou
d) l'un de X₁ et X₂ est C=NOR₂₀ et l'autre est CH₂.

3. Composé selon la revendication 1, dans lequel X₁ est NR₁₄ et X₂ est O.

4. Composé selon la revendication 1, dans lequel R₁₀ est du chlore, un groupe halogénoalkyle en C₁-C₄ ou un groupe halogénovinyle.

5. Composé selon la revendication 1, dans lequel R₃ est un halogène ou un groupe halogénoalkyle en C₁-C₆, halogénoalcényle en C₂-C₆ ou halogénoalcynyle en C₂-C₆.

6. Composé selon la revendication 1, choisi dans la classe formée par les suivants :
4-(α,α,α,4-tétrafluoro-N-méthyl-*m*-toluidino)-6-[(α,α,α,4-tétrafluoro-m-tolyl)oxy]pyrimidine ;
4-(α,α,α,4-trifluoro-N-méthyl-méthyl-*m*-toluidino)-6-[(α,α,α-trifluoro-*m*-tolyl)oxy]pyrimidine ;
4-[(α,α,α,4-tétrafluoro-*m*-tolyl)oxy]-6-(α,α,α-trifluoro-N-méthyl-m-toluidino)pyrimidine ;
4-(3,4-dichlorophényloxy)-6-(α,α,α-trifluoro-N-méthyl-*m*-toluidino)pyrimidine ;
4-[(4-chloro-α,α,α-trifluoro-*m*-tolyl)oxy]-6-(α,α,α-trifluoro-N-méthyl-m-toluidino)pyrimidine ;
4-(α,α,α-4-tétrafluoro-N-méthyl-*m*-toluidino)-6-[(α,α,α-trifluoro-*m*-tolyl)oxy]pyrimidine ;
4-[(4-chloro-α,α,α-trifluoro-*m*-tolyl)oxy]-6-(α,α,α,4-tétrafluoro-N-méthyl-m-toluidino)pyrimidine ;
4-(4-chloro-α,α,α-trifluoro-N-méthyl*-m*-toluidino)-6-[(α,α,α,4-tétrafluoro-*m*-tolyl)oxy]pyrimidine ;
4-(3,4-dichloro-N-méthylanilino)-6-[(α,α,α-trifluoro-*m*-tolyl)oxy]pyrimidine ;
4-(3,4-dichloro-N-méthylanilino)-6-[(α,α,α,4-tétrafluoro-*m*-tolyl)oxy]pyrimidine ;
4-(3,4-dichloro-N-méthylanilino)-6-(3,4-dichlorophénoxy)pyrimidine ;
chlorhydrate de 4-(α,α,α,4-tétrafluoro-*m*-toluidino)-6-[*m*-(trifluorométhyl)benzyl]pyrimidine ;
chlorhydrate de 4-(3,4-dichlorobenzyl)-6-(α,α,α,4-tétrafluoro-*m*-toluidino)pyrimidine ;
chlorhydrate de 4-(4-chloro-α,α,α-trifluoro-m-toluidino)-6-[*m*-(trifluorométhyl)benzyl]pyrimidine ;
chlorhydrate de 4-[*m*-(trifluorométhyl)benzyl]-6-(α,α,α-trifluoro-*m*-toluidino)pyrimidine ;
4-(α,α,α,4-tétrafluoro-N-méthyl-*m*-toluidino)-6-[*m*-(trifluorométhyl)benzyl]pyrimidine ;
4-(4-chloro-α,α,α-trifluoro-N-méthyl-*m*-toluidino)-6-(3,4-dichlorobenzyl)pyrimidine ;
4-(3,4-dichlorobenzyl)-6-(α,α,α,4-tétrafluoro-N-méthyl-*m*-toluidino)pyrimidine ;
4-(4-chloro-α,α,α-trifluoro-*m*-toluidino)-6-(3,4-dichlorobenzyl)pyrimidine ;
4-(3,4-dichlorobenzyl)-6-[N-(diéthoxyméthyl)-α,α,α,4-tétrafluoro-*m*-toluidino]pyrimidine
4-(4-chloro-α,α,α-trifluoro-N-méthyl-*m*-toluidino)-6-[*m*-(trifluorométhyl)benzyl]pyrimidine ;
4-(3,4-dichlorobenzyl)-6-(α,α,α-trifluoro-*m*-toluidino)pyrimidine ;
4-(3,4-dichlorobenzyl)-6-(α,α,α-trifluoro-N-méthyl-*m*-toluidino)pyrimidine ;
6-(α,α,α,4-tétrafluoro-*m*-toluidino)-4-[4-fluoro-3-(trifluorométhyl)benzyl]pyrimidine ;
4-[4-fluoro-3-(trifluorométhyl)benzyl]-6-(α,α,α,4-tétrafluoro-N-méthyl-*m*-toluidino)pyrimidine ;
4-(4-chloro-α,α,α-trifluoro-*m*-toluidino)-6-[4-fluoro-3-(trifluorométhyl)benzyl]pyrimidine ;
4-(4-chloro-α,α,α-trifluoro-N-méthyl-*m*-toluidino)-6- [4-fluoro-3-(trifluorométhyl)benzyl]pyrimidine ;
4-[*m*-(trifluorométhyl)benzyl]-6-[(α,α,α-trifluoro-*m*-tolyl)oxy]pyrimidine ;
4- (*p*-chloro-α,α-diméthylbenzyl)-6-[(α,α,α,4-tétrafluoro-*m*-tolyl)oxy]pyrimidine ;
4-(*p*-chloro-α,α-diméthylbenzyl)-6-[(α,α,α,4-trifluoro-*m*-tolyl)oxy]pyrimidine ;
4-[1-(*p*-chlorophényl)cyclopropyl]-6-[(α,α,α-trifluoro-*m*-tolyl)oxy]pyrimidine ;
4-[1-(*p*-chlorophényl)cyclopropyl]-6-[(α,α,α,4-tétrafluoro-*m*-tolyl)oxy]pyrimidine ;
6-[(α,α,α,4-tétrafluoro-*m*-tolyl)oxy]-4-pyrimidinyl-α,α,α,4-trifluoro-*m*-tolyl-cétone ;
4- [α,α-diméthyl-*m*- (trifluorométhyl)benzyl]-6-[(α,α,α-trifluoro-*m*-tolyl)oxy]pyrimidine ;
4-[α,α-diméthyl-*m*-(trifluorométhyl)benzyl]-6-[(α,α,α,4-tétrafluoro-*m*-tolyl)oxy]pyrimidine ; et
(*E*) - et (*Z*) - O-méthyloxime de 6-[*m*-(trifluorométhyl)benzyl]-4-pyrimidinyl-α,α,α-trifluoro-*m*-tolyl-cétone.

7. Composé selon la revendication 1, dans lequel R₁ est un groupe alkylthio en C₁-C₆, halogénoalkylthio en C₁-C₆, alkylsulfinyle en C₁-C₆, halogénoalkylsulfinyle en C₁-C₆, alkylsulfoxyle en C₁-C₆, halogénoalkylsulfoxyle en C₁-C₆ ou NR₁₂R₁₃ ; et l'un de X₁ et X₂ est NR₁₄ et l'autre est O.

8. Procédé de lutte contre des acariens ou des insectes, qui consiste à mettre en contact ledit acarien ou insecte ou leurs habitats ou leurs zones ou sites de reproduction avec une quantité à effet acaricide ou insecticide d'un composé décrit dans l'une quelconque des revendications 1 à 7.

9. Composition qui comprend un support acceptable en agriculture et une quantité à effet pesticide d'un composé décrit dans l'une quelconque des revendications 1 à 7.

10. Procédé pour la préparation d'un composé de formule I dans lequel X₁, X₂, R₁, R₂, R₃, R₄, R₅, R₆, R₇, R₈, R₉, R₁₀ et R₁₁ sont tels que définis dans l'une quelconque des revendications 1 à 7, qui comprend les étapes suivantes :
i) faire réagir un composé de formule IV 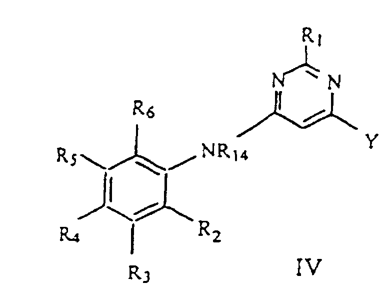 avec un phénol de formule V ou un thiophénol de formule V¹ 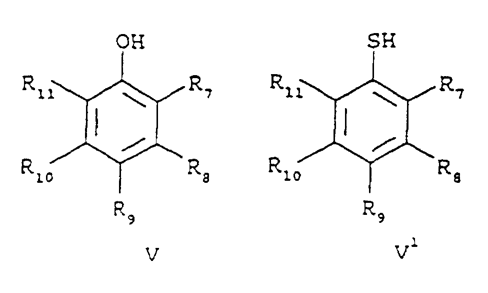 pour obtenir un composé de formule I dans lequel X₁ est NR₁₄ et X₂ est O ou S ;
ii) faire réagir un composé de formule IV^{a} 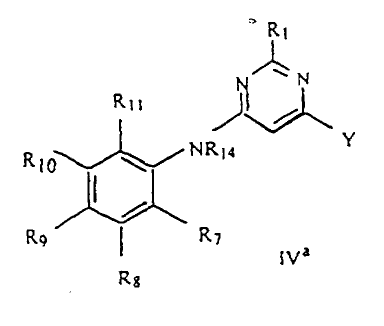 avec un phénol de formule V^{a} ou un thiophénol de formule V^{1a} 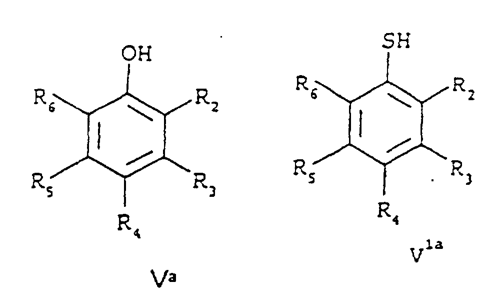 pour obtenir un composé de formule I dans lequel X₁ est O ou S et X₂ est NR₁₄ ;
iii) faire réagir un composé de formule X 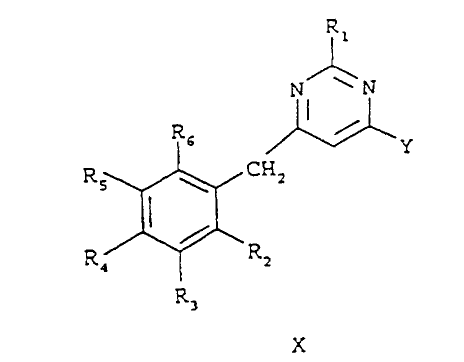 dans laquelle Y est un halogène, avec le dérivé d'aniline approprié de formule XI 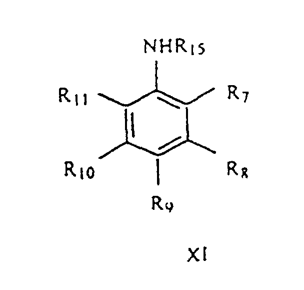 pour obtenir un composé de formule I dans lequel X₁ est CH₂ et X₂ est NR₁₅ ;
iv) faire réagir un composé de formule X^{a} 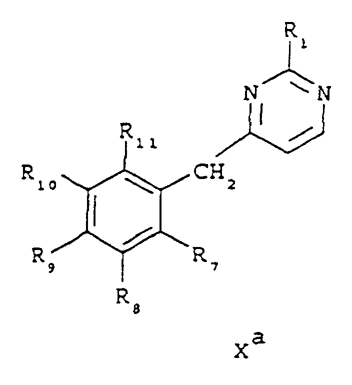 dans laquelle Y est un halogène, avec le dérivé d'aniline approprié de formule XI^{a} 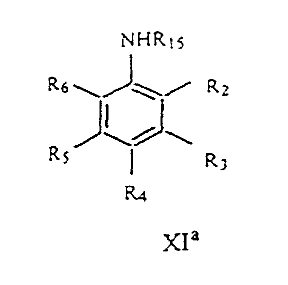 pour obtenir un composé de formule I dans lequel X₁ est NR₁₅ et X₂ est CH₂ ;
v) réduire un composé de formule XVIII 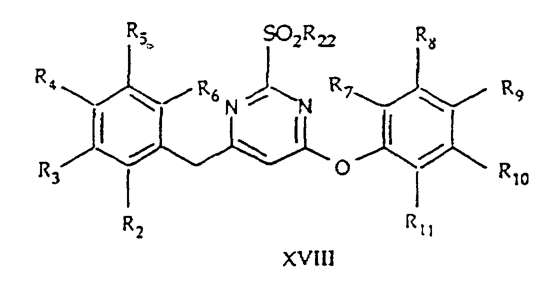 pour obtenir un composé de formule I dans lequel X₁ est CR₁₈R₁₉, R₁₈ et R₁₉ sont tous deux l'hydrogène, X₂ est O et R₁ est l'hydrogène ;
vi) réduire un composé de formule XVIII^{a} 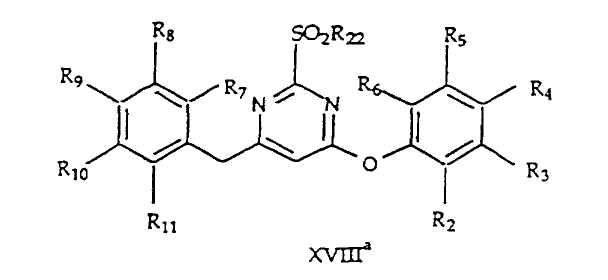 pour obtenir un composé de formule I dans lequel X₁ est O, X₂ est CR₁₈R₁₉, R₁₈ et R₁₉ sont tous deux l'hydrogène et R₁ est l'hydrogène ;
vii) faire réagir un composé de formule XVIII avec une amine de formule XIX :
NHR₁₂R₁₃ XIX
pour obtenir un composé de formule I dans lequel X₁ est CR₁₈R₁₉, R₁₈ et R₁₉ sont tous deux l'hydrogène, X₂ est O et R₁ est NR₁₂R₁₃ ;
viii) faire réagir un composé de formule XVIII^{a} avec une amine de formule XIX, telle que définie ci-dessus, pour obtenir un composé de formule I dans lequel X₁ est O, X₂ est CR₁₈R₁₉, R₁₈ et R₁₉ sont tous deux l'hydrogène et R₁ est NR₁₂R₁₃ ;
ix) faire réagir un composé de formule XXIV 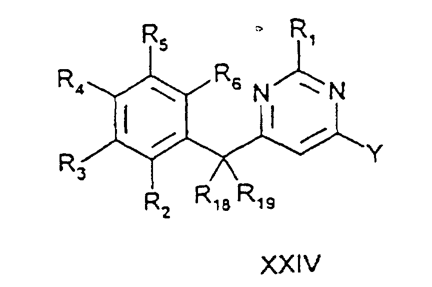 dans laquelle Y est un halogène, avec un phénol de formule V 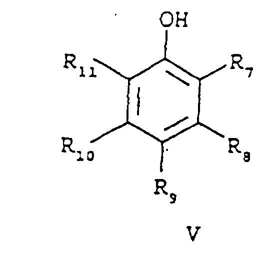 pour obtenir un composé de formule I dans lequel X₁ est CR₁₈R₁₉, X₂ est O et R₁₈ et R₁₉ sont choisis chacun parmi les groupes alkyle en C₁-C₆, alcényle en C₂-C₆ et alcynyle en C₂-C₆ qui sont tous facultativement substitués par un ou plusieurs halogènes identiques ou différents, ou bien R₁₈ et R₁₉ peuvent former ensemble un carbocycle qui est facultativement substitué par un ou plusieurs halogènes identiques ou différents ;
x) faire réagir un composé de formule XXIV^{a} 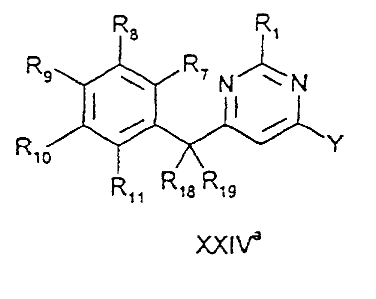 dans laquelle Y est un halogène, avec un phénol de formule V^{a} 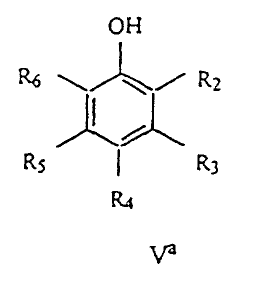 pour obtenir un composé dans lequel X₁ est O, X₂ est CR₁₈R₁₉, et R₁₈ et R₁₉ sont choisis chacun parmi les groupes alkyle en C₁-C₆, alcényle en C₂-C₆ et alcynyle en C₂-C₆ qui sont tous facultativement substitués par un ou plusieurs halogènes identiques ou différents, ou bien R₁₈ et R₁₉ peuvent former ensemble un carbocycle qui est facultativement substitué par un ou plusieurs halogènes identiques ou différents ;
xi) oxyder un composé de formule I dans lequel X₁ est CH₂ et X₂ est O en le composé correspondant dans lequel X₁ est CO ;
xii) oxyder un composé de formule I dans lequel X₂ est CH₂ et X₁ est O en le composé correspondant dans lequel X₂ est CO ;
xiii) oximer un composé de formule XXIX 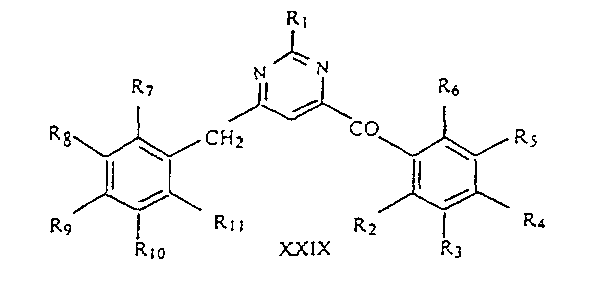 en l'oxime correspondante pour obtenir un composé de formule I dans lequel X₁ est C=NOR₂₀ et X₂ est CH₂ ; ou
xiv) oximer un composé de formule XXIX^{a} 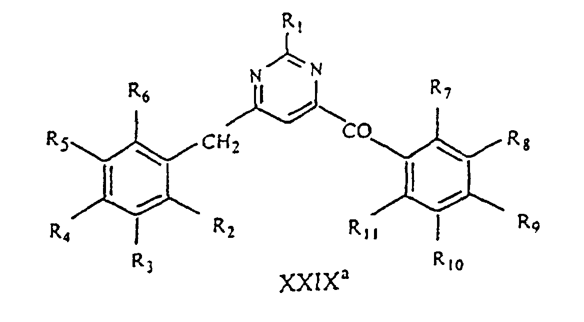 en l'oxime correspondante pour obtenir un composé de formule I dans lequel X₁ est CH₂ et X₂ est C=NOR₂₀,
